# EUROPEAN PATENT SPECIFICATION

(11) **EP 0 871 702 B1**
(45) Date of publication and mention of the grant of the patent: **25.05.2005**
(21) Application number: 95944314.4
(22) Date of filing: 05.12.1995
(51) Int. Cl.: C12N 1/20, C12N 15/12, C12N 15/19, A61K 38/19, A61K 38/20, C07K 14/52, C07K 14/54, C07K 16/24

(54) **CYTOKINE DESIGNATED LERK-7**
CYTOKIN LERK-7
CYTOKINE DESIGNEE PAR LERK-7

(30) Priority: 06.12.1994 US 351025; 01.03.1995 US 396946
(43) Date of publication of application: 21.10.1998
(73) Proprietor: Immunex Corporation, Thousand Oaks, CA 91320 (US)
(72) Inventor: CERRETTI, Douglas P., Seattle, WA 98133 (US)
(74) Representative: Bassil, Nicholas Charles
(86) International application number: PCT/US1995/015781
(87) International publication number: WO 1996/017925

(56) References cited:
- WO-A-96/13518
- WO-A-96/23000
- DAVIS S ET AL: "LIGANDS FOR EPH-RELATED RECEPTOR TYROSINE KINASES THAT REQUIRE MEMBRANE ATTACHMENT OR CLUSTERING FOR ACTIVITY" SCIENCE, vol. 266, 4 November 1994, pages 816-819, XP002038222
- HWAI-JONG CHENG ET AL: "Identification and cloning of ELF-1, a developmentally expressed ligand for the Mek4 and Sek Receptor Tyrosine kinases" CELL, vol. 79, October 1994, pages 157-168, XP002107086 NA US
- C.J. KOZLOSKY ET AL: "Ligands for the receptor tyrosine kinases hek and elk: Isolation of cDNAs encoding a family of proteins" ONCOGENE, vol. 10, no. 2, 19 January 1995, pages 299-306, XP002106260
- WINSLOW J W ET AL: "CLONING OF AL-1, A LIGAND FOR AN EPH-RELATED TYROSINE KINASE RECEPTOR INVOLVED IN AXON BUNDLE FORMATION," NEURON, vol. 14, 1 May 1995, pages 973-981, XP000566892
- ONCOGENE, Volume 9, Number 11, issued November 1994, FLETCHER et al., "LERK-2, a Binding Protein for the Receptor-Tyrosine Kinase ELK, is Evolutionarily Conserved and Expressed in a Developmentally Regulated Pattern", pages 3241-3247.
- ONCOGENE, Volume 8, issued 1993, BOHME et al., "PCR Mediated Detection of a New Human Receptor-Tyrosine-Kinase, HEK 2", pages 2857-2862.
- THE EMBO JOURNAL, Volume 13, Number 16, issued August 1994, BECKMANN et al., "Molecular Characterization of a Family of Ligands for Eph-related Tyrosine Kinase Receptors", pages 3757-3762.
- PROC. NATL. ACAD. SCI. U.S.A., Volume 89, issued March 1992, WICKS et al., "Molecular Cloning of HEK, the Gene Encoding a Receptor Tyrosine Kinase Expressed by Human Lymphoid Tumor Cell Lines", pages 1611-1615.

### SEQUENCE LISTING

(1) GENERAL INFORMATION:
   (i) APPLICANT:
      (A) NAME: Immunex Corporation
      (B) STREET: 51 University Street
      (C) CITY: Seattle
      (D) STATE: Washington 98101
      (E) COUNTRY: U.S.A
      (F) POSTAL CODE (ZIP): none
      (G) TELEPHONE: 2065870430
      (H) TELEFAX: 2065870606
      (I) TELEX: none
   (ii) TITLE OF INVENTION: Cytokine designated Lerk-7
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA: APPLICATION NUMBER: EP 95944314.4
(2) INFORMATION FOR SEQ ID NO: 1:
   (i) SEQUENCE CHARACTERISTICS:
      (A) LENGTH: 555 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Lerk-6
   (ix) FEATURE:
      (A) NAME/KEY: CDS
      (B) LOCATION:1..552
   (xi) SEQUENCE DESCRIPTION: SEQ ID NO: 1:
   GCC CGG GCC AAC GCT GAC CGA TAC GCA GTC TAC TGG AAC CGT AGC AAC          48
   Ala Arg Ala Asn Ala Asp Arg Tyr Ala Val Tyr Trp Asn Arg Ser Asn
     1               5                  10                  15
   CCC AGG TTT CAG GTG AGC GCT GTG GGT GAT GGC GGC GGC TAT ACC GTG          96
   Pro Arg Phe Gln Val Ser Ala Val Gly Asp Gly Gly Gly Tyr Thr Val
                20                  25                  30
   GAG GTG AGC ATC AAC GAC TAC CTG GAT ATC TAC TGC CCA CAC TAC GGG         144
   Glu Val Ser Ile Asn Asp Tyr Leu Asp Ile Tyr Cys Pro His Tyr Gly
            35                  40                  45
   GCG CCG CTG CCC CCG GCT GAG CGC ATG GAG CGG TAC ATC CTG TAC ATG      192
   Ala Pro Leu Pro Pro Ala Glu Arg Met Glu Arg Tyr Ile Leu Tyr Met
        50                  55                  60
   GTG AAT GGT GAG GGC CAC GCC TCC TGT GAC CAC CGG CAG CGA GGC TTC      240
   Val Asn Gly Glu Gly His Ala Ser Cys Asp His Arg Gln Arg Gly Phe
    65                  70                  75                  80
   AAG CGC TGG GAA TGC AAC CGG CCC GCA GCG CCC GGG GGA CCC CTC AAG      288
   Lys Arg Trp Glu Cys Asn Arg Pro Ala Ala Pro Gly Gly Pro Leu Lys
                    85                  90                  95
   TTC TCA GAG AAG TTC CAA CTC TTC ACC CCC TTT TCC CTG GGC TTT GAG      336
   Phe Ser Glu Lys Phe Gln Leu Phe Thr Pro Phe Ser Leu Gly Phe Glu
               100                 105                 110
   TTC CGG CCT GGC CAC GAA TAC TAC TAC ATC TCT GCC ACA CCT CCC AAC      384
   Phe Arg Pro Gly His Glu Tyr Tyr Tyr Ile Ser Ala Thr Pro Pro Asn
           115                 120                 125
   CTC GTG GAC CGA CCC TGC CTG CGA CTC AAG GTT TAT GTG CGT CCA ACC      432
   Leu Val Asp Arg Pro Cys Leu Arg Leu Lys Val Tyr Val Arg Pro Thr
       130                 135                 140
   AAT GAG ACC CTG TAT GAG GCT CCA GAG CCC ATC TTC ACC AGT AAC AGC      480
   Asn Glu Thr Leu Tyr Glu Ala Pro Glu Pro Ile Phe Thr Ser Asn Ser
   145                 150                 155                 160
   TCC TGC AGC GGC CTG GGT GGC TGC CAC CTC TTC CTC ACC ACC GTC CCT      528
   Ser Cys Ser Gly Leu Gly Gly Cys His Leu Phe Leu Thr Thr Val Pro
                   165                 170                 175
   GTG CTG TGG TCC CTT CTG GGC TCC TAG                                  555
   Val Leu Trp Ser Leu Leu Gly Ser
               180
(2) INFORMATION FOR SEQ ID NO: 2:
   (i) SEQUENCE CHARACTERISTICS:
      (A) LENGTH: 184 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Ala Arg Ala Asn Ala Asp Arg Tyr Ala Val Tyr Trp Asn Arg Ser Asn
    1               5                  10                  15
   Pro Arg Phe Gln Val Ser Ala Val Gly Asp Gly Gly Gly Tyr Thr Val
               20                  25                  30
   Glu Val Ser Ile Asn Asp Tyr Leu Asp Ile Tyr Cys Pro His Tyr Gly
           35                  40                  45
   Ala Pro Leu Pro Pro Ala Glu Arg Met Glu Arg Tyr Ile Leu Tyr Met
       50                  55                  60
   Val Asn Gly Glu Gly His Ala Ser Cys Asp His Arg Gln Arg Gly Phe
   65                  70                  75                  80
   Lys Arg Trp Glu Cys Asn Arg Pro Ala Ala Pro Gly Gly Pro Leu Lys
                   85                  90                  95
   Phe Ser Glu Lys Phe Gln Leu Phe Thr Pro Phe Ser Leu Gly Phe Glu
               100                 105                 110
   Phe Arg Pro Gly His Glu Tyr Tyr Tyr Ile Ser Ala Thr Pro Pro Asn
           115                 120                 125
   Leu Val Asp Arg Pro Cys Leu Arg Leu Lys Val Tyr Val Arg Pro Thr
       130                 135                 140
   Asn Glu Thr Leu Tyr Glu Ala Pro Glu Pro Ile Phe Thr Ser Asn Ser
   145                 150                 155                 160
   Ser Cys Ser Gly Leu Gly Gly Cys His Leu Phe Leu Thr Thr Val Pro
                   165                 170                 175
   Val Leu Trp Ser Leu Leu Gly Ser
               180
(2) INFORMATION FOR SEQ ID NO: 3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 3:
   CTTTAGTGAG GGTTAATCTA TAG                                            23
(2) INFORMATION FOR SEQ ID NO: 4:
   (i) SEQUENCE CHARACTERISTICS:
      (A) LENGTH: 687 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: hulerk7
   (ix) FEATURE:
      (A) NAME/KEY: sig_peptide
      (B) LOCATION:1..60
   (ix) FEATURE:
      (A) NAME/KEY: CDS
      (B) LOCATION:1..687
   (ix) FEATURE:
      (A) NAME/KEY: mat_peptide
      (B) LOCATION:61..684
   (xi) SEQUENCE DESCRIPTION: SEQ ID NO: 4:
   ATG TTG CAC GTG GAG ATG TTG ACG CTG GTG TTT CTG GTG CTC TGG ATG          48
   Met Leu His Val Glu Met Leu Thr Leu Val Phe Leu Val Leu Trp Met
   -20                -15                 -10                  -5
   TGT GTG TTC AGC CAG GAC CCG GGC TCC AAG GCC GTC GCC GAC CGC TAC          96
   Cys Val Phe Ser Gln Asp Pro Gly Ser Lys Ala Val Ala Asp Arg Tyr
                       1               5                   10
   GCT GTC TAC TGG AAC AGC AGC AAC CCC AGA TTC CAG AGG GGT GAC TAC         144
   Ala Val Tyr Trp Asn Ser Ser Asn Pro Arg Phe Gln Arg Gly Asp Tyr
           15                  20                  25
   CAT ATT GAT GTC TGT ATC AAT GAC TAC CTG GAT GTT TTC TGC CCT CAC         192
   His Ile Asp Val Cys Ile Asn Asp Tyr Leu Asp Val Phe Cys Pro His
       30                  35                  40
   TAT GAG GAC TCC GTC CCA GAA GAT AAG ACT GAG CGC TAT GTC CTC TAC         240
   Tyr Glu Asp Ser Val Pro Glu Asp Lys Thr Glu Arg Tyr Val Leu Tyr
   45                  50                  55                  60
   ATG GTG AAC TTT GAT GGC TAC AGT GCC TGC GAC CAC ACT TCC AAA GGG         288
   Met Val Asn Phe Asp Gly Tyr Ser Ala Cys Asp His Thr Ser Lys Gly
                       65                  70                  75
   TTC AAG AGA TGG GAA TGT AAC CGG CCT CAC TCT CCA AAT GGA CCG CTG         336
   Phe Lys Arg Trp Glu Cys Asn Arg Pro His Ser Pro Asn Gly Pro Leu
               80                  85                  90
   AAG TTC TCT GAA AAA TTC CAG CTC TTC ACT CCC TTT TCT CTA GGA TTT         384
   Lys Phe Ser Glu Lys Phe Gln Leu Phe Thr Pro Phe Ser Leu Gly Phe
           95                  100                 105
   GAA TTC AGG CCA GGC CGA GAA TAT TTC TAC ATC TCC TCT GCA ATC CCA         432
   Glu Phe Arg Pro Gly Arg Glu Tyr Phe Tyr Ile Ser Ser Ala Ile Pro
       110                 115                 120
   GAT AAT GGA AGA AGG TCC TGT CTA AAG CTC AAA GTC TTT GTG AGA CCA         480
   Asp Asn Gly Arg Arg Ser Cys Leu Lys Leu Lys Val Phe Val Arg Pro
   125                 130                 135                 140
   ACA AAT AGC TGT ATG AAA ACT ATA GGT GTT CAT GAT CGT GTT TTC GAT         528
   Thr Asn Ser Cys Met Lys Thr Ile Gly Val His Asp Arg Val Phe Asp
                   145                 150                 155
   GTT AAC GAC AAA GTA GAA AAT TCA TTA GAA CCA GCA GAT GAC ACC GTA         576
   Val Asn Asp Lys Val Glu Asn Ser Leu Glu Pro Ala Asp Asp Thr Val
               160                 165                 170
   CAT GAG TCA GCC GAG CCA TCC CGC GGC GAG AAC GCG GCA CAA ACA CCA         624
   His Glu Ser Ala Glu Pro Ser Arg Gly Glu Asn Ala Ala Gln Thr Pro
           175                 180                 185
   AGG ATA CCC AGC CGC CTT TTG GCA ATC CTA CTG TTC CTC CTG GCG ATG         672
   Arg Ile Pro Ser Arg Leu Leu Ala Ile Leu Leu Phe Leu Leu Ala Met
       190                 195                 200
   CTT TTG ACA TTA TAG                                                     687
   Leu Leu Thr Leu
   205
(2) INFORMATION FOR SEQ ID NO: 5:
   (i) SEQUENCE CHARACTERISTICS:
      (A) LENGTH: 228 amino acids
      (B) TYPE: amino acid
      (D) TOPOLOGY: linear
   (ii) MOLECULE TYPE: protein
   (xi) SEQUENCE DESCRIPTION: SEQ ID NO: 5:
   Met Leu His Val Glu Met Leu Thr Leu Val Phe Leu Val Leu Trp Met
   -20                 -15                 -10                  -5
   Cys Val Phe Ser Gln Asp Pro Gly Ser Lys Ala Val Ala Asp Arg Tyr
                     1               5                  10
   Ala Val Tyr Trp Asn Ser Ser Asn Pro Arg Phe Gln Arg Gly Asp Tyr
            15                  20                  25
   His Ile Asp Val Cys Ile Asn Asp Tyr Leu Asp Val Phe Cys Pro His
        30                  35                  40
   Tyr Glu Asp Ser Val Pro Glu Asp Lys Thr Glu Arg Tyr Val Leu Tyr
    45                  50                  55                  60
   Met Val Asn Phe Asp Gly Tyr Ser Ala Cys Asp His Thr Ser Lys Gly
                    65                  70                  75
   Phe Lys Arg Trp Glu Cys Asn Arg Pro His Ser Pro Asn Gly Pro Leu
                80                  85                  90
   Lys Phe Ser Glu Lys Phe Gln Leu Phe Thr Pro Phe Ser Leu Gly Phe
            95                 100                 105
   Glu Phe Arg Pro Gly Arg Glu Tyr Phe Tyr Ile Ser Ser Ala Ile Pro
       110                 115                 120
   Asp Asn Gly Arg Arg Ser Cys Leu Lys Leu Lys Val Phe Val Arg Pro
   125                 130                 135                 140
   Thr Asn Ser Cys Met Lys Thr Ile Gly Val His Asp Arg Val Phe Asp
                   145                 150                 155
   Val Asn Asp Lys Val Glu Asn Ser Leu Glu Pro Ala Asp Asp Thr Val
               160                 165                 170
   His Glu Ser Ala Glu Pro Ser Arg Gly Glu Asn Ala Ala Gln Thr Pro
           175                 180                 185
   Arg Ile Pro Ser Arg Leu Leu Ala Ile Leu Leu Phe Leu Leu Ala Met
       190                 195                 200
   Leu Leu Thr Leu
   205
(2) INFORMATION FOR SEQ ID NO: 6:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (vii) IMMEDIATE SOURCE:
      (B) CLONE: FLAG peptide
   (xi) SEQUENCE DESCRIPTION: SEQ ID NO: 6:
   Asp Tyr Lys Asp Asp Asp Asp Lys
   1               5
(2) INFORMATION FOR SEQ ID NO: 7:
   (i) SEQUENCE CHARACTERISTICS:
      (A) LENGTH: 705 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vii) IMMEDIATE SOURCE:
      (B) CLONE: hIgG1Fc
   (ix) FEATURE:
      (A) NAME/KEY: CDS
      (B) LOCATION:1..699
   (xi) SEQUENCE DESCRIPTION: SEQ ID NO: 7:
   GAG CCC AGA TCT TGT GAC AAA ACT CAC ACA TGC CCA CCG TGC CCA GCA        48
   Glu Pro Arg Ser Cys Asp Lys Thr His Thr Cys Pro Pro Cys Pro Ala
    1               5                  10                  15
   CCT GAA CTC CTG GGG GGA CCG TCA GTC TTC CTC TTC CCC CCA AAA CCC        96
   Pro Glu Leu Leu Gly Gly Pro Ser Val Phe Leu Phe Pro Pro Lys Pro
                   20                  25                  30
   AAG GAC ACC CTC ATG ATC TCC CGG ACC CCT GAG GTC ACA TGC GTG GTG       144
   Lys Asp Thr Leu Met Ile Ser Arg Thr Pro Glu Val Thr Cys Val Val
               35                  40                  45
   GTG GAC GTG AGC CAC GAA GAC CCT GAG GTC AAG TTC AAC TGG TAC GTG       192
   Val Asp Val Ser His Glu Asp Pro Glu Val Lys Phe Asn Trp Tyr Val
           50                  55                  60
   GAC GGC GTG GAG GTG CAT AAT GCC AAG ACA AAG CCG CGG GAG GAG CAG       240
   Asp Gly Val Glu Val His Asn Ala Lys Thr Lys Pro Arg Glu Glu Gln
    65                  70                  75                  80
   TAC AAC AGC ACG TAC CGG GTG GTC AGC GTC CTC ACC GTC CTG CAC CAG       288
   Tyr Asn Ser Thr Tyr Arg Val Val Ser Val Leu Thr Val Leu His Gln
                   85                  90                  95                 305
   GAC TGG CTG AAT GGC AAG GAC TAC AAG TGC AAG GTC TCC AAC AAA GCC       336
   Asp Trp Leu Asn Gly Lys Asp Tyr Lys Cys Lys Val Ser Asn Lys Ala
               100                 105                 110
   CTC CCA GCC CCC ATG CAG AAA ACC ATC TCC AAA GCC AAA GGG CAG CCC       384
   Leu Pro Ala Pro Met Gln Lys Thr Ile Ser Lys Ala Lys Gly Gln Pro
               115                 120                 125
   CGA GAA CCA CAG GTG TAC ACC CTG CCC CCA TCC CGG GAT GAG CTG ACC       432
   Arg Glu Pro Gln Val Tyr Thr Leu Pro Pro Ser Arg Asp Glu Leu Thr
           130                 135                 140
   AAG AAC CAG GTC AGC CTG ACC TGC CTG GTC AAA GGC TTC TAT CCC AGG       480
   Lys Asn Gln Val Ser Leu Thr Cys Leu Val Lys Gly Phe Tyr Pro Arg
   145                 150                 155                 160
   CAC ATC GCC GTG GAG TGG GAG AGC AAT GGG CAG CCG GAG AAC AAC TAC       528
   His Ile Ala Val Glu Trp Glu Ser Asn Gly Gln Pro Glu Asn Asn Tyr
                   165                 170                 175                385
   AAG ACC ACG CCT CCC GTG CTG GAC TCC GAC GGC TCC TTC TTC CTC TAC       576
   Lys Thr Thr Pro Pro Val Leu Asp Ser Asp Gly Ser Phe Phe Leu Tyr
               180                 185                 190
   AGC AAG CTC ACC GTG GAC AAG AGC AGG TGG CAG CAG GGG AAC GTC TTC       624
   Ser Lys Leu Thr Val Asp Lys Ser Arg Trp Gln Gln Gly Asn Val Phe
           195                 200                 205
   TCA TGC TCC GTG ATG CAT GAG GCT CTG CAC AAC CAC TAC ACG CAG AAG       672
   Ser Cys Ser Val Met His Glu Ala Leu His Asn His Tyr Thr Gln Lys
           210                 215                 220
   AGC CTC TCC CTG TCT CCG GGT AAA TGA ACTAGT                            705
   Ser Leu Ser Leu Ser Pro Gly Lys
   225                 230
(2) INFORMATION FOR SEQ ID NO: 8:
   (i) SEQUENCE CHARACTERISTICS:
      (A) LENGTH: 232 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Glu Pro Arg Ser Cys Asp Lys Thr His Thr Cys Pro Pro Cys Pro Ala
     1               5                  10                  15
   Pro Glu Leu Leu Gly Gly Pro Ser Val Phe Leu Phe Pro Pro Lys Pro
                20                  25                  30
   Lys Asp Thr Leu Met Ile Ser Arg Thr Pro Glu Val Thr Cys Val Val
            35                  40                  45
   Val Asp Val Ser His Glu Asp Pro Glu Val Lys Phe Asn Trp Tyr Val
        50                  55                  60
   Asp Gly Val Glu Val His Asn Ala Lys Thr Lys Pro Arg Glu Glu Gln
    65                  70                  75                  80
   Tyr Asn Ser Thr Tyr Arg Val Val Ser Val Leu Thr Val Leu His Gln
                    85                  90                  95
   Asp Trp Leu Asn Gly Lys Asp Tyr Lys Cys Lys Val Ser Asn Lys Ala
                   100                 105                 110
   Leu Pro Ala Pro Met Gln Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Arg
   145                 150                 155                 160
   His Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230

## Description

### BACKGROUND OF THE INVENTION

Proteins known as the receptor tyrosine kinases have an intrinsic kinase activity that is activated upon ligand binding. This class of proteins is characterized by conserved structural motifs within the catalytic domains (Hanks et al., *Science,* 242:42, 1988) and can be subdivided into families based on structural features of the regions N-terminal to the catalytic domain.

The *eph* family of receptors, named after the first member isolated (Hirai et al., *Science* 238:1717, 1987) is the largest subfamily of receptor tyrosine kinases. Among the members of this family are chicken cek4 (Sajjadi et al. *New Biol.* 3:769, 1991) and cek5 (Pasquale, E.B., *Cell Regulation* 2:523, 1991); murine mek4 (Sajjadi et al., *supra),* bsk (Zhou et al., *J. Neurosci. Res.,* 37:129, 1994), nuk (Henkemeyer et al., *Oncogene* 9:1001, 1994), and sek (Gilardi-Hebenstreit et al., *Oncogene* 7:2499, 1992); rat elk (Letwin et al., *Oncogene* 3:621, 1988; Lhotak et al., *Mol. Cell. Biol*. 11:2496, 1991), eek (Chan et al., *Oncogene* 6:1057, 1991), ehk-1 and ehk-2 (Maisonpierre et al., *Oncogene* 8:3277, 1993); and human hek (Boyd et al., *J. Biol. Chem.,* 267:3262, 1992; Wicks et al., *PNAS USA,* 89:1611, 1992), hek2 (Bohme et al., *Oncogene* 8:2857, 1993), eck (Lindberg et al. *Mol. Cell. Biol*. 10:6316, 1990), and erk (Chan et al., *supra*).

The proteins of this subfamily are related not only in their cytoplasmic domains, but also in their extracellular domains, which are 41 to 68% identical. Interestingly, the tissue distributions of these various receptors are diverse. For example, expression of elk mRNA has been reported to be limited to testis and brain (Lhotak et al., *supra*), whereas eck is found not only in these same two tissues but in lung, intestine, kidney, spleen, ovary, and skin as well. Because most eph-related receptor tyrosine kinases are primarily expressed in the brain, it has been postulated that these receptors and their ligands may be involved in the growth, differentiation, and survival of neurons.

The cell-surface glycoprotein designated hek (human *eph*/*elk*-like kinase) was purified by Boyd et al. (*J. Biol. Chem.,* 267:3262, 1992). A monoclonal antibody immunoreactive with hek was used to study hek expression on a number of human cell types (Boyd et al., *supra*). Hek antigen was detected on the human pre-B cell leukemia cell line LK63 (the cell line employed as the immunogen against which the antibody was raised) and the human T-cell leukemia cell line, JM. The Raji B lymphoma cell line showed weak hek antigen expression, and the remaining cell lines tested (both normal and tumor cell lines, among which were hemopoietic cell lines that included pre-B and T-cell lines) were consistently negative. Of the normal and tumor tissue biopsy specimens that were also tested for hek antigen expression, none of the normal tissues was positive and only a very low proportion of hemopoietic tumors was positive.

Expression of hek transcripts in the above-described LK63 and JM cell lines, as well as the human T-cell leukemia cell line HSB-2, has been demonstrated by northern blot analysis (Wicks et al., *Proc. Natl. Acad. Sci. USA,* 89:1611, 1992). Nucleotide and amino acid sequences for an isolated hek cDNA clone are also presented in Wicks et al., *supra.*

A partial clone of the cell surface protein designated elk was first discovered in a rat brain cDNA expression library that was screened for proteins expressing tyrosine kinase activity (Letwin et al., *Oncogene* 3:621, 1988). Later, a composite sequence spanning the entire elk coding region was derived from partial clones isolated from a rat brain cDNA library and a rat cerebellar brain library using the partial clone as a probe (Lhotak et al., *Mol. Cell. Biol*. 11:2496, 1991).

Those ligands that have been identified for the receptor tyrosine kinases are a diverse group of proteins that affect the growth, differentiation, and survival of cells expressing the receptors. Due to the homology of the receptors in the eph family, a given ligand for one specific receptor may also bind other receptors. Ligands for hek and elk have been isolated, as discussed in more detail below.

Identification of additional ligands for hek and elk that may exist would prove useful in investigating the nature of cellular processes regulated by signaling through these receptors. If enhancement or inhibition of a particular biological signal mediated through these receptors is desired, it is advantageous to identify each of the proteins that may play a role in transduction of such signals. Further, it is known that certain proteins can bind to receptors without initiating signal transduction, including interleukin-1 receptor antagonist protein (Eisenberg et al., *Nature* 343:341, 1990; Hannum et al., *Nature* 343:336, 1990; and Carter et al., *Nature* 344:633, 1990). Identification of additional proteins that bind hek or elk is also desirable in order to determine whether such proteins function as antagonists.

### SUMMARY OF THE INVENTION

The present invention is directed to a novel cytokine designated Lerk-7. Purified Lerk-7 proteins are provided herein, along with isolated DNAs encoding Lerk-7, expression vectors comprising the Lerk-7 DNA, and host cells transformed with the expression vectors. Processes for producing Lerk-7 include culturing such transformed host cells under conditions that promote expression of Lerk-7.

The Lerk-7 polypeptides bind to the cell surface receptors known as hek, elk, and eck, which are described above. The invention also encompasses antibodies that specifically bind Lerk-7 polypeptides.

### DETAILED DESCRIPTION OF THE INVENTION

A novel cytokine designated Lerk-7 is provided herein. This cytokine binds to the receptor tyrosine kinases known as elk, hek, and eck.

The present invention encompasses DNA encoding Lerk-7, expression vectors comprising the Lerk-7 DNA, and host cells transformed with the expression vectors. A method for producing Lerk-7 polypeptides comprises culturing the transformed host cells under conditions conducive to expression of Lerk-7, and recovering the expressed Lerk-7. Purified Lerk-7 polypeptides in both soluble and membrane-bound form are disclosed.

Lerk-7 polypeptides or immunogenic fragments thereof may be employed as immunogens to generate antibodies that are immunoreactive therewith. In one embodiment of the invention, the antibodies are monoclonal antibodies.

A cDNA encoding human Lerk-7 has been isolated from a human fetal brain cDNA library, as described in example 1. The nucleotide sequence of the coding region of this Lerk-7 cDNA is presented in SEQ ID NO:4, and the amino acid sequence encoded thereby is presented in SEQ ID NO:5. This Lerk-7 protein comprises an N-terminal signal peptide (amino acids -20 to -1 OF SEQ ID NO:5), an extracellular receptor-binding domain (amino acids 1 to 133), a spacer region (amino acids 134 to 183), and a C-terminal stretch of hydrophobic residues (amino acids 194-208).

Lerk-7 is predicted to be anchored to the cell surface via glycosylphosphatidylinositol (GPI) linkage. GPI membrane anchors, including the chemical structure and processing thereof, are described in Ferguson, M. and A. Williams, *Ann. Rev. Biochem*., *57*:285, 1988. When initially expressed, certain proteins comprise a C-terminal hydrophobic domain that contains signals for GPI anchoring. A cleavage site is located upstream, often about 10-12 amino acids upstream of the N-terminus of the hydrophobic domain. Post translational processing includes cleavage of the protein at this cleavage site. A GPI anchor attaches to the newly exposed C-terminal amino acid of the processed, mature protein. Thus, when Lerk-7 proteins are expressed in cells that recognize GPI anchoring signals, the full length amino acid sequence of SEQ ID NO:5 represents a precursor form of the protein.

The predicted GPI attachment site in the Lerk-7 protein is the asparagine residue at position 183. After cleavage of the protein (during post translational processing), this asparagine residue becomes the C-terminus of the processed protein. A GPI moiety attaches to this asparagine residue. This prediction of the likely cleavage site is based on consensus sequences found in other GPI-anchored proteins. It is possible that cleavage occurs elsewhere upstream of the hydrophobic region.

The present invention provides both cell membrane-bound and soluble (secreted) forms of Lerk-7. Soluble Lerk-7 polypeptides include the receptor-binding domain of a native Lerk-7, but lack the GPI signal that would cause retention of the polypeptide on a cell membrane. Soluble Lerk-7 polypeptides encompassed by the invention retain the ability to bind the hek or elk receptor. Soluble Lerk-7 may also include the spacer region or part of the hydrophobic domain, provided that the soluble Lerk-7 protein can be secreted.

Soluble Lerk-7 may be identified (and distinguished from its non-soluble membrane-bound counterparts) by separating intact cells expressing a Lerk-7 polypeptide from the culture medium, e.g., by centrifugation, and assaying the medium (supernatant) for the presence of the desired protein. The presence of Lerk-7 in the medium indicates that the protein was secreted from the cells and thus is a soluble form of the desired protein.

Soluble forms of Lerk-7 possess certain advantages over the membrane-bound form of the protein. Purification of the protein from recombinant host cells is facilitated, since the soluble proteins are secreted from the cells. Further, soluble proteins are generally more suitable for certain applications, e.g., for intravenous administration.

Soluble Lerk-7 proteins are truncated at the C-terminus. Deletion of the hydrophobic domain is believed to be sufficient to prevent GPI anchoring of the protein to the cell membrane, although the protein may be further truncated to delete the amino acid that constitutes the GPI attachment site. Examples of soluble human Lerk-7 polypeptides include, but are not limited to, polypeptides truncated at the C-terminus so that the C-terminal amino acid is any of those between or including the residues at positions 133 and 193 of SEQ ID NO:5.

In particular embodiments, soluble Lerk-7 polypeptides include those comprising amino acids 1-133, 1-182, 1-185, or 1-193 of SEQ ID NO:5. To illustrate one embodiment, an expression vector encoding a recombinant fusion protein comprising amino acids -20 to 185 of SEQ ID NO:5, followed by a peptide encoded by a vector multiple cloning site sequence, followed by an Fc region polypeptide (derived from an antibody) was prepared. This fusion protein was secreted from the host cells in which it was expressed, and exhibited biological activity, as evidenced by binding to eck. In another alternative, the soluble polypeptide is a fragment of the Lerk-7 receptor binding domain that retains the ability to bind elk or hek.

When initially expressed within a host cell, soluble Lerk-7 polypeptides advantageously comprise the native signal peptide or one of the heterologous signal peptides described below that is functional within the host cells employed. Isolated DNA sequences encoding soluble Lerk-7 proteins are encompassed by the present invention.

Lerk-7 fragments, including soluble polypeptides, may be prepared by any of a number of conventional techniques. A DNA sequence encoding a truncated Lerk-7 may be chemically synthesized using known techniques. DNA fragments also may be produced by restriction endonuclease digestion of a full length cloned DNA sequence, and isolated by electrophoresis on agarose gels. Oligonucleotides that reconstruct the 5' or 3'-terminus of a DNA fragment to a desired point may be utilized. Linkers containing restriction endonuclease cleavage site(s) may be employed to insert the desired DNA fragment into an expression vector. The well known polymerase chain reaction (PCR) procedure also may be employed to amplify a DNA fragment encoding a particular protein fragment. Primers that define the desired termini of the DNA fragment are employed in the PCR. As a further alternative, known mutagenesis techniques may be employed to insert a stop codon at a desired point, e.g., immediately downstream of the codon for the last amino acid of the receptor-binding domain.

Other proteins that bind to both hek and elk have been discovered, and are designated Lerk-1 through Lerk-6 (ligands of the eph-related kinases). Lerks 2 and 5 are type 1 transmembrane proteins, while Lerks 1, 3, 4, and 6 are anchored to the cell membrane by GPI linkage. The percent identity of the amino acid sequences of these six proteins ranges from 30 to 59%, and the proteins each have four conserved cysteine residues.

Holzman et al. (*Mol. Cell. Biol*. 10:5830, 1990) reported the cloning of cDNA for a protein called B61. The ability of B61 to bind to elk and to hek was discovered subsequently, and the B61 protein was given the alternative designation Lerk-1 (Beckmann et al., *EMBO J. 13*:3757, 1994). B61 has also been reported to be a ligand for the above-described receptor tyrosine kinase known as eck (Bartley et al., *Nature* 368:558, 1994).

Lerk-2, also known as elk ligand, is described in PCT application WO 94/11384. Both Lerk-3 and Lerk-4 are described in U.S. Patent 5,516,658. Lerk-5 is described in U.S. Patent 6,303,769.

Lerk-6 DNA and proteins are described in U.S. Patent 5,919,905. A murine Lerk-6 cDNA clone designated λ13 was isolated from an 11.5 day murine embryonic cDNA library. A substantially complete DNA sequence of the coding region of the clone λ13 cDNA, and the amino acid sequence encoded thereby, are presented herein in SEQ ID NO: and SEQ ID NO:2, respectively. The open-reading frame within this sequence encodes a protein of 184 amino acids. The first amino acid of SEQ ID NO:2 (Ala) is believed to be located at or very near the native N-terminus. A cell lysate containing clone λ13 DNA (the Lerk-6 cDNA in λgt10) was deposited with the American Type Culture Collection, Rockville, MD, USA on July 15, 1994, and assigned accession number ATCC 75829.

The human Lerk-7 cDNA of the present invention was discovered during an attempt to isolate cDNA encoding the human homolog of murine Lerk-6. A mouse Lerk-6 DNA fragment was used as a probe to screen a human cDNA library in an attempt to clone human Lerk-6. Surprisingly, an isolated cDNA clone encoded a novel protein, the Lerk-7 of the present invention.

The percent identity of the human Lerk-7 amino acid sequence of SEQ ID NO:5 with the full length amino acid sequence of various other proteins is as follows, wherein "h" represents human, "m" represents mouse, and "r" represents rat.:

| | |
|---|---|
| h Lerk-1 | 45.771 |
| h Lerk-2 | 27.602 |
| r Lerk-2 | 25.676 |
| m Lerk-2 | 26.126 |
| h Lerk-3 | 42.342 |
| h Lerk-4 | 42.000 |
| h Lerk-5 | 25.792 |
| m Lerk-5 | 25.114 |
| m Lerk-6 | 55.330 |

As used herein, the term "Lerk-7" refers to a genus of polypeptides that are substantially homologous to the human Lerk-7 protein described in example 1. The polypeptides preferably comprise an amino acid sequence that is at least 80% identical, and more preferably at least 90% identical, to the amino acid sequence of SEQ ID NO:5, as further described below. The Lerk-7 polypeptides are capable of binding to the above-described receptors designated hek and elk. Certain uses of Lerk-7 flow from this ability to bind to elk or hek, as described in more detail below.

Lerk-7 also binds to the receptor designated eck (for epithelial cell kinase), which is described in Lindberg and Hunter (*Mol. Cell. Biol. 10*:6316, 1990). eck is expressed predominantly in cell lines of epithelial origin and in tissues that contain a significant proportion of epithelial cells (Lindberg and Hunter, *supra*). Cells expressing eck include both normal and cancer cell types (Lindberg and Hunter, *supra*). Additional uses of Lerk-7 flow from its ability to bind eck, as described below.

Human Lerk-7 nucleic acids and proteins are provided herein. Also within the scope of the present invention are Lerk-7 nucleic acids and proteins derived from other mammalian species that include but are not limited to murine, bovine, porcine, equine, or various primate species.

The Lerk-7 polypeptides provided herein include variants of native Lerk-7 polypeptides that retain a biological activity of a native Lerk-7. The term "Lerk-7 variants" as used herein refers to polypeptides that are substantially homologous to native Lerk-7, but which have an amino acid sequence different from that of a native Lerk-7 because of one or more deletions, insertions or substitutions. Likewise, the Lerk-7-encoding DNAs of the present invention include variants that differ from a native Lerk-7 DNA sequence because of one or more deletions, insertions or substitutions, but that encode a biologically active Lerk-7 polypeptide. The term "biologically active" as it refers to Lerk-7, indicates that the Lerk-7 is capable of binding to hek or to elk.

The variant DNA or amino acid sequences preferably are at least 80% identical to a native Lerk-7 sequence, most preferably at least 90% identical. The percent identity may be determined, for example, by comparing sequence information using the GAP computer program, version 6.0 described by Devereux et al. (*Nucl. Acids Res.* 12:387, 1984) and available from the University of Wisconsin Genetics Computer Group (UWGCG). The preferred default parameters for the GAP program include: (1) a unary comparison matrix (containing a value of 1 for identities and 0 for non-identities) for nucleotides, and the weighted comparison matrix of Gribskov and Burgess, *Nucl. Acids Res. 14*:6745, 1986, as described by Schwartz and Dayhoff, eds., *Atlas of Protein Sequence and Structure*, National Biomedical Research Foundation, pp. 353-358, 1979; (2) a penalty of 3.0 for each gap and an additional 0.10 penalty for each symbol in each gap; and (3) no penalty for end gaps.

Variant Lerk-7 polypeptides thus include Lerk-7 fragments that retain the ability to bind hek or elk. Examples of truncated Lerk-7 polypeptides within the scope of the present invention are soluble (secreted) polypeptides.

Additional embodiments of variant amino acid sequences are those comprising conservative substitutions, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. A Lerk-7 containing amino acid substitution(s) is considered herein to be conservatively substituted when the resulting non-native polypeptide retains a desired biological activity of native Lerk-7.

The invention further includes Lerk-7 polypeptides with or without associated native-pattern glycosylation. Lerk-7 expressed in yeast or mammalian expression systems (e.g., COS-7 cells) may be similar to or significantly different from a native Lerk-7 polypeptide in molecular weight and glycosylation pattern, depending upon the choice of expression system. Expression of Lerk-7 polypeptides in bacterial expression systems, such as *E. coli,* provides non-glycosylated molecules.

N-glycosylation sites in the Lerk-7 extracellular domain can be modified to preclude glycosylation, allowing expression of a more homogeneous, reduced carbohydrate analog in mammalian and yeast expression systems. N-glycosylation sites in eukaryotic polypeptides are characterized by an amino acid triplet Asn-X-Y, wherein X is any amino acid except Pro and Y is Ser or Thr. The human Lerk-7 protein of SEQ ID NO:5 comprises one such triplet, at amino acids 17-19 of SEQ ID NO:5. Appropriate substitutions, additions or deletions to the nucleotide sequence encoding these triplets will result in prevention of attachment of carbohydrate residues to the Asn side chain. Alteration of a single nucleotide, chosen so that Asn is replaced by a different amino acid, for example, is sufficient to inactivate an N-glycosylation site. Known procedures for inactivating N-glycosylation sites in proteins include those described in U.S. Patent 5,071,972 and EP 276,846.

In another example of variants, sequences encoding Cys residues that are not essential for biological activity can be altered to cause the Cys residues to be deleted or replaced with other amino acids, preventing formation of incorrect intramolecular disulfide bridges upon renaturation. Cysteine residues corresponding to the four cysteines that are conserved among the Lerk proteins are found at positions 42, 70, 82, and 131 of SEQ ID NO:5. To maintain the biological activity of the native protein, these four cysteines desirably remain unaltered.

Other variants are prepared by modification of adjacent dibasic amino acid residues to enhance expression in yeast systems in which KEX2 protease activity is present. EP 212,914 discloses the use of site-specific mutagenesis to inactivate KEX2 protease processing sites in a protein. KEX2 protease processing sites are inactivated by deleting, adding or substituting residues to alter Arg-Arg, Arg-Lys, and Lys-Arg pairs to eliminate the occurrence of these adjacent basic residues. Lys-Lys pairings are considerably less susceptible to KEX2 cleavage, and conversion of Arg-Lys or Lys-Arg to Lys-Lys represents a conservative and preferred approach to inactivating KEX2 sites. The human Lerk-7 contains two KEX2 protease processing sites, at amino acids 78-79 and 128-129 of SEQ ID NO:5.

Naturally occurring Lerk-7 variants or alleles are also encompassed by the invention. Examples of such variants are proteins that result from alternate mRNA splicing events or from proteolytic cleavage of the Lerk-7 protein, wherein the elk- or hek-binding property is retained. Alternate splicing of mRNA may yield a truncated but biologically active Lerk-7 protein, such as a naturally occurring soluble form of the protein, for example. Variations attributable to proteolysis include, for example, differences in the Nor C-termini upon expression in different types of host cells, due to proteolytic removal of one or more terminal amino acids from the Lerk-7 protein (generally from 1-5 terminal amino acids).

Regarding the foregoing discussion of the signal peptide and various domains of Lerk-7 protein, the skilled artisan will recognize that the above-described boundaries of such regions of the protein are approximate. For example, although computer programs that predict the site of cleavage of a signal peptide are available, cleavage can occur at sites other than those predicted. Further, it is recognized that a protein preparation can comprise a mixture of protein molecules having different N-terminal amino acids, due to cleavage of the signal peptide at more than one site. In addition, post-translational processing can vary according to the particular expression system employed. Thus, the N- or C-terminal amino acid of a recombinant protein may vary according to the type of host cells in which the protein was expressed, for example.

Variants and derivatives of native Lerk-7 proteins may be prepared by mutation of nucleotide sequences encoding native Lerk-7 polypeptides. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, oligonucleotide-directed site-specific mutagenesis procedures can be employed to introduce a desired mutation. Methods for making such alterations include those disclosed by Walder et al. (*Gene* 42:133, 1986); Bauer et al. (*Gene* 37:73, 1985); Craik (*BioTechniques*, January 1985, 12-19); Smith et al. (*Genetic Engineering: Principles and Methods,* Plenum Press, 1981); Kunkel (*Proc. Natl. Acad. Sci. USA* 82:488, 1985); Kunkel et al. (*Methods in Enzymol*. 154:367, 1987); and U.S. Patent Nos. 4,518,584 and 4,737,462.

Lerk-7 may be modified to create Lerk-7 derivatives by forming covalent or aggregative conjugates with other chemical moieties, such as glycosyl groups, lipids, phosphate, acetyl groups and the like. Covalent derivatives of Lerk-7 may be prepared by linking the chemical moieties to functional groups on Lerk-7 amino acid side chains or at the N-terminus or C-terminus of a Lerk-7 polypeptide or the extracellular domain thereof. Other derivatives of Lerk-7 within the scope of this invention include covalent or aggregative conjugates of Lerk-7 polypeptides with other proteins or polypeptides, such as by synthesis in recombinant culture as N-terminal or C-terminal fusions.

Lerk-7 polypeptide fusions can comprise peptides added to facilitate purification and identification of Lerk-7. Such peptides include, for example, poly-His or the antigenic identification peptides described in U.S. Patent No. 5,011,912 and in Hopp et al., *BiolTechnology* 6:1204, 1988. One such peptide is the Flag® peptide, Asp-Tyr-Lys-Asp-Asp-Asp-Asp-Lys, which is highly antigenic and provides an epitope reversibly bound by a specific monoclonal antibody, enabling rapid assay and facile purification of expressed recombinant protein. A murine hybridoma designated 4E11 produces a monoclonal antibody that binds the Flag® peptide in the presence of certain divalent metal cations, as described in U.S. Patent 5,011,912. The 4E11 hybridoma cell line has been deposited with the American Type Culture Collection under accession no. HB 9259. Monoclonal antibodies that bind the Flag® peptide are available from Eastman Kodak Co., Scientific Imaging Systems Division, New Haven, Connecticut.

Due to the known degeneracy of the genetic code, wherein more than one codon can encode the same amino acid, a DNA sequence can vary from that shown in SEQ ID NO:4 and still encode a Lerk-7 protein having the amino acid sequence of SEQ ID NO:5. Such variant DNA sequences may result from silent mutations (e.g., occurring during PCR amplification), or may be the product of deliberate mutagenesis of a native sequence. The present invention thus provides isolated DNA sequences selected from native Lerk-7 DNA sequences (e.g., cDNA comprising the nucleotide sequence presented in SEQ ID NO:4) and DNA that is degenerate as a result of the genetic code to a native Lerk-7 DNA sequence.

Lerk-7 variants possessing the ability to bind hek or elk may be identified by any suitable assay. Biological activity of a Lerk-7 variant may be determined, for example, by assaying for the variant's ability to compete with a native Lerk-7 for binding to hek or elk (i.e. competitive binding assays).

Competitive binding assays can be performed following conventional methodology. Reagents that may be employed in competitive binding assays include radiolabeled, soluble Lerk-7 and intact hek/elk-expressing cells. For example, radiolabeled native Lerk-7 can be used to compete with a Lerk-7 variant for binding to cell surface-bound hek or elk. Instead of intact cells, one could substitute a soluble hek/Fc or elk/Fc fusion protein bound to a solid phase through the interaction of Protein A or Protein G with the Fc moiety. Chromatography columns that contain Protein A and Protein G include those available from Pharmacia Biotech, Inc.. Piscataway, NJ. Another type of competitive binding assay utilizes radiolabeled soluble hek or elk, such as a soluble hek/Fc or elk/Fc fusion protein, and intact cells expressing Lerk-7. Qualitative results can be obtained by competitive autoradiographic plate binding assays, or Scatchard plots may be utilized to generate quantitative results.

The biological activity of a native Lerk-7 protein or fragment thereof expressed in a particular expression system can be confirmed using competition binding assays. For example, the Lerk-7 may be assayed for the ability to compete with one of the other Lerk proteins (e.g., Lerk-6) for binding to elk or hek.

Lerk-7 also binds to the receptor known as eck (Lindberg and Hunter, *Mol. Cell. Biol. 10*:6316, 1990). It is possible that the Lerk-7 of the present invention will bind to other receptors of the *eph* family (see the background section). Such binding can be analyzed using a suitable assay analogous to those described above and in examples 4 and 7.

Uses of Lerk-7 that flow from the ability to bind elk, hek, and eck include, but are not limited to, the following. Lerk-7 finds use as a protein purification reagent. Lerk-7 polypeptides may be attached to a solid support material and used to purify hek, elk, or eck proteins by affinity chromatography. In certain embodiments, Lerk-7 fragments or fusion proteins (e.g., Lerk-7/Fc fusions) containing the receptor-binding domain of Lerk-7 are attached to the solid support. Lerk-7 polypeptides may be attached to a solid support material by conventional procedures. As one example, chromatography columns containing functional groups that will react with functional groups on amino acid side chains of proteins are available (Pharmacia Biotech, Inc., Piscataway, NJ). Lerk-7/Fc fusion proteins can be attached to Protein A- or Protein G-containing chromatography columns through interaction with the Fc moiety.

Lerk-7 proteins also find use in purifying cells that express hek, elk, or eck on the cell surface. The Lerk-7 (or fragments of fusions thereof) is bound to a solid phase such as a column chromatography matrix or a similar suitable substrate. For example, magnetic microspheres can be coated with Lerk-7 and held in an incubation vessel through a magnetic field. Suspensions of cell mixtures containing hek/elk/eck-expressing cells are contacted with the solid phase having Lerk-7 thereon. Cells expressing hek or elk or eck on the cell surface bind to the fixed Lerk-7, and unbound cells then are washed away. This affinity-binding method is useful for purifying or identifying such hek/elk/eck-expressing cells.

Methods of releasing positively selected cells from the solid phase are known in the art and encompass, for example, the use of enzymes. Suitable enzymes are non-toxic and non-injurious to the cells and are preferably directed to cleaving the hek or elk or eck proteins, thereby freeing the resulting cell suspension from the "foreign" Lerk-7 material.

The purified cell population, especially if obtained from fetal tissue, then may be used to repopulate mature (adult) tissues. For example, neural cells expressing elk may be isolated by the foregoing procedure, then administered to a mammal afflicted with a neurodegenerative disorder.

Alternatively, mixtures of cells suspected of containing hek/elk⁺ cells first can be incubated with biotinylated Lerk-7. Incubation periods are typically at least one hour in duration to ensure sufficient binding to hek/elk The resulting mixture then is passed through a column packed with avidin-coated beads, whereby the high affinity of biotin for avidin provides the binding of the cell to the beads. Use of avidin-coated beads is known in the art. See Berenson, et al. *J. Cell. Biochem*., 10D:239 (1986). Washing of unbound material and the release of the bound cells is performed using conventional methods.

Lerk-7 thus can be employed in separating or purifying the above-described cell types expressing hek or elk. Lerk-7 also finds use in identifying additional types of cells that express hek or elk on the cell surface. Lerk-7 can be conjugated to a detectable moiety such as a radionuclide to detect hek/elk-expressing cells. As one example, radiolabeling with ¹²⁵I can be performed by any of several standard methodologies that yield a functional ¹²⁵I-Lerk-7 molecule labeled to high specific activity. Another detectable moiety such as an enzyme that can catalyze a colorimetric or fluorometric reaction, biotin or avidin may be used. Cells to be tested for hek/elk-expression can be contacted with labeled Lerk-7. After incubation, unbound labeled Lerk-7 is removed and the presence or absence of the detectable moiety on the cells is determined.

Lerk-7 proteins also may be employed to measure the biological activity of elk or hek proteins in terms of their binding affinity for Lerk-7. Lerk-7 proteins thus find use as reagents that may be employed by those conducting "quality assurance" studies, e.g., to monitor shelf life and stability of elk or hek protein under different conditions. As additional examples, Lerk-7 is used in determining whether biological activity is retained after modification of an elk or hek protein (e.g., chemical modification, truncation, mutation, etc.). The biological activity of an elk or hek protein thus can be ascertained before it is used in a research study, or possibly in the clinic, for example.

To illustrate. Lerk-7 is employed in a binding affinity study to measure the biological activity of an elk protein that has been stored at different temperatures, or produced in different cell types. The binding affinity of the modified elk protein for Lerk-7 is compared to that of an unmodified elk protein to detect any adverse impact of the modifications on biological activity of elk. Likewise, the biological activity of a hek protein can be assessed using Lerk-7.

Lerk-7 polypeptides also find use as carriers for delivering agents attached thereto to cells bearing the elk or hek cell surface receptor. Expression of hek antigen has been reported for certain leukemic cell lines, including the human T-cell leukemia cell line designated JM and the human pre-B cell leukemia cell line designated LK63 (Boyd et al., *J. Biol. Chem.* 267:3262, 1992, and Wicks et al., *Proc. Natl. Acad. Sci. USA,* 89:1611, 1992). Lerk-7 proteins thus can be used to deliver diagnostic or therapeutic agents to these cells (or to other cell types found to express hek or elk on the cell surface) in *in vitro* or *in vivo* procedures.

One example of such use is to expose a hek⁺ leukemic cell line to a therapeutic agent/Lerk-7 conjugate to assess whether the agent exhibits cytotoxicity toward the leukemic cells. A number of different therapeutic agents attached to Lerk-7 may be included in an assay to detect and compare the cytotoxic effect of the agents on the leukemic cells. Lerk-7/diagnostic agent conjugates may be employed to detect the presence of hek⁺ cells *in vitro* or *in vivo.*

Diagnostic and therapeutic agents that may be attached to a Lerk-7 polypeptide include, but are not limited to, drugs, toxins, radionuclides, chromophores, enzymes that catalyze a colorimetric or fluorometric reaction, and the like, with the particular agent being chosen according to the intended application. Examples of drugs include those used in treating various forms of cancer, e.g., nitrogen mustards such as L-phenylalanine nitrogen mustard or cyclophosphamide, intercalating agents such as cis-diaminodichloroplatinum, antimetabolites such as 5-fluorouracil, vinca alkaloids such as vincristine, and antibiotics such as bleomycin, doxorubicin, daunorubicin, and derivatives thereof. Among the toxins are ricin, abrin, diphtheria toxin, *Pseudomonas aeruginosa* exotoxin A, ribosomal inactivating proteins, mycotoxins such as trichothecenes, and derivatives and fragments (e.g., single chains) thereof. Radionuclides suitable for diagnostic use include, but are not limited to, ¹²³I, ¹³¹I, ^{99m}Tc, ¹¹¹In, and ⁷⁶Br. Radionuclides suitable for therapeutic use include, but are not limited to, ¹³¹I, ²¹¹At, ⁷⁷Br, ¹⁸⁶Re, ¹⁸⁸Re, ²¹²Pb, ²¹²Bi, ¹⁰⁹Pd, ⁶⁴Cu, and ⁶⁷Cu.

Such agents may be attached to the Lerk-7 by any suitable conventional procedure. Lerk-7, being a protein, comprises functional groups on amino acid side chains that can be reacted with functional groups on a desired agent to form covalent bonds, for example. Alternatively, the protein or agent may be derivatized to generate or attach a desired reactive functional group. The derivatization may involve attachment of one of the bifunctional coupling reagents available for attaching various molecules to proteins (Pierce Chemical Company, Rockford, Illinois). A number of techniques for radiolabeling proteins are known. Radionuclide metals may be attached to Lerk-7 by using a suitable bifunctional chelating agent, for example.

Conjugates comprising Lerk-7 and a suitable diagnostic or therapeutic agent (preferably covalently linked) are thus prepared. The conjugates are administered or otherwise employed in an amount appropriate for the particular application.

Another use of the Lerk-7 of the present invention is as a research tool for studying the role that Lerk-7, in conjunction with elk or hek, may play in growth or differentiation of cells bearing the elk or hek receptor. The Lerk-7 polypeptides of the present invention also may be employed in *in vitro* assays for detection of elk or Lerk-7 or the interactions thereof. Likewise, Lerk-7 finds use in assays for hek or the interaction of Lerk-7 with hek. The possibility that hek plays a role in tumorigenesis has been suggested (Boyd et al., *supra*). The Lerk-7 protein is useful for investigating what effect binding of Lerk-7 to hek may have on tumorigenesis.

While certain uses of Lerk-7 are illustrated herein with respect to the elk-binding or hek-binding properties of Lerk-7, it is to be understood that analogous uses arise from the ability of Lerk-7 to bind to eck. Examples of the cancer cell types that express eck are the human epithelial carcinoma cell line HeLa, a human epidermal carcinoma cell line designated A431 (Lindberg and Hunter, *supra*), melanoma cell lines (Easty et al., *Cancer Research 55*:2528, 1995) and the human colon adeno carcinoma cell line HT29. Lerk-7 can be used to deliver diagnostic or therapeutic agents to such cells, as discussed above with respect to hek-bearing cells.

Lerk-7 nucleic acids may be employed in detecting defects of the Lerk-7 gene, e.g., in an *in vitro* assay conducted on DNA samples derived from an individual to be tested for such a defect. DNA of the present invention may be employed in replacing defective Lerk-7 genes, e.g., in gene therapy procedures.

As discussed above, when various rat tissues were analyzed for elk mRNA, transcripts were detected only in brain and testis (Lhotak et al., *supra*). Binding of Lerk-7 to elk on neural tissue may exert a neuroprotective or neurotrophic effect.

Lerk-7 finds use as a tissue culture reagent. A Lerk-7 protein can be added to neurons cultured *in vitro* to enhance the viability or prolong the lifespan of the cultured neurons, thus facilitating research studies, and possible clinical treatment of neural tissue.

The above-described Lerk-2 protein has been found to exert a neurotrophic effect on hippocampal neurons, and to protect the neurons against glutamate-mediated excitotoxicity. Lerk-7 likewise may exhibit neuroprotective or neurotrophic properties. Lerk-7 thus may find use in a method for treating disorders of neural tissue, involving contacting the neural tissue with Lerk-7. Such disorders include injury or neurologic diseases, either chronic or acute. The use of Lerk-7 in preparing a medicament for treating disease or injury of neural tissue is contemplated herein.

Compositions comprising an effective amount of a Lerk-7 polypeptide of the present invention, in combination with other components such as a suitable diluent, carrier, or excipient, are provided herein. Lerk-7 can be formulated according to known methods used to prepare pharmaceutically useful compositions. Lerk-7 can be combined in admixture, either as the sole active material or with other known active materials, with pharmaceutically suitable diluents (e.g., saline, Tris-HCl, acetate, and phosphate buffered solutions), preservatives (e.g., thimerosal, benzyl alcohol, parabens), emulsifiers, solubilizers, adjuvants and/or carriers. Suitable carriers and their formulations are described in Remington's Pharmaceutical Sciences, 16th ed. 1980, Mack Publishing Company.

In addition, such compositions can contain Lerk-7 complexed with polyethylene glycol (PEG), metal ions, or incorporated into polymeric compounds such as polyacetic acid, polyglycolic acid, hydrogels, dextran, etc., or incorporated into liposomes, microemulsions, micelles, unilamellar or multilamellar vesicles, erythrocyte ghosts or spheroblasts. Such compositions will influence the physical state, solubility, stability, rate of *in vivo* release, and rate of *in vivo* clearance of Lerk-7, and are thus chosen according to the intended application. As one alternative, Lerk-7 is GPI-linked to a substrate, wherein the substrate is composed of physiologically acceptable material having a chemical composition suitable for attachment of Lerk-7 through GPI linkages. The Lerk-7-bearing substrate may be surgically implanted. Lerk-7 can also be conjugated to antibodies against tissue-specific receptors, ligands or antigens, or coupled to ligands of tissue-specific receptors.

Such compositions may contain a Lerk-7 polypeptide in any form described herein, such as native proteins, variants, derivatives, biologically active fragments or oligomers. In one embodiment, the composition comprises a soluble Lerk-7 polypeptide.

Lerk-7 can be administered in any suitable manner, e.g., topically, parenterally, or by inhalation. The term "parenteral" includes injection, e.g., by subcutaneous, intravenous, or intramuscular routes, also including localized injection, e.g., at a site of disease or injury. Sustained release from implants is also comtemplated. Suitable dosages and desired drug concentrations contained in the compositions may vary depending upon many factors, including the intended use, patient's body weight and age, and route of administration. Preliminary doses can be determined according to animal tests, and the scaling of dosages for human administration can be performed according to art-accepted practices.

### Oligomeric Forms of Lerk-7

Encompassed by the present invention are Lerk-7 polypeptides in the form of oligomers, such as covalently-linked or non-covalently-linked dimers, trimers, or higher oligomers. Such oligomers may be naturally occurring or produced by recombinant DNA technology. Oligomers may be linked by disulfide bonds formed between cysteine residues on different Lerk-7 polypeptides.

Oligomers of the present invention include, but are not limited to, oligomers comprising from two to four Lerk-7 polypeptides. In one embodiment, the Lerk-7 polypeptides are soluble.

As one alternative, a Lerk-7 oligomer is prepared using polypeptides derived from immunoglobulins. Preparation of fusion proteins comprising certain heterologous polypeptides fused to various portions of antibody-derived polypeptides (including the Fc domain) has been described, e.g., by Ashkenazi et al. (*PNAS USA* 88:10535, 1991), Byrn et al. (*Nature* 344:677, 1990), and Hollenbaugh et al. (*Current Protocols in Immunology,* Suppl. 4, 1992, pp. 10.19.1-10.19.11), hereby incorporated by reference. One embodiment of the present invention is directed to a Lerk-7 dimer created by fusing Lerk-7 to the Fc region of an antibody (e.g., IgGl) in a manner that does not interfere with binding of Lerk-7 to the hek or elk ligand-binding domain. The Fc polypeptide preferably is fused to the C-terminus of a soluble Lerk-7 comprising only the extracellular domain.

A gene fusion encoding the Lerk-7/Fc fusion protein is inserted into an appropriate expression vector. Lerk-7/Fc fusion proteins are expressed in host cells transformed with the recombinant expression vector, and allowed to assemble much like antibody molecules, whereupon interchain disulfide bonds form between Fc polypeptides, yielding divalent Lerk-7. If fusion proteins are made with both heavy and light chains of an antibody, it is possible to form a Lerk-7 oligomer with as many as four Lerk-7 extracellular regions.

Provided herein are fusion proteins comprising a Lerk-7 polypeptide fused to an Fc polypeptide derived from an antibody. DNA encoding such fusion proteins, as well as dimers containing two fusion proteins joined via disulfide bonds between the Fc moieties thereof, are also provided. The term "Fc polypeptide" as used herein includes native and mutein forms of polypeptides derived from the Fc region of an antibody. Truncated forms of such polypeptides containing the hinge region that promotes dimerization are also included. One suitable Fc polypeptide, described in PCT application WO 93/10151, is a single chain polypeptide extending from the N-terminal hinge region to the native C-terminus. A mutein of this Fc polypeptide is described in example three below. The mutein exhibits reduced affinity for Fc receptors.

One method for preparing oligomeric Lerk-7 involves use of a leucine zipper. Leucine zipper domains are peptides that promote oligomerization of the proteins in which they are found. Leucine zippers were originally identified in several DNA-binding proteins (Landschulz et al., *Science 240*:1759, 1988), and have since been found in a variety of different proteins. Among the known leucine zippers are naturally occurring peptides and derivatives thereof that dimerize or trimerize. Examples of leucine zipper domains useful for producing soluble oligomeric proteins are described in PCT application WO94/10308. In one embodiment, recombinant fusion proteins comprising a soluble Lerk-7 polypeptide fused to a peptide that dimerizes or trimerizes in solution are expressed in suitable host cells. Soluble Lerk-7 oligomers are recovered from the culture medium.

Alternatively, the oligomer is a fusion protein comprising multiple Lerk-7 polypeptides, with or without spacer peptides between the Lerk-7 moieties. Such fusion proteins are produced by recombinant DNA technology. In one embodiment, a fusion protein comprises two or more soluble Lerk-7 polypeptides, separated by peptide linkers.

The oligomers have the property of bivalent, trivalent, etc. binding sites for elk or hek. Further, the above-described fusion proteins comprising Fc moieties (and oligomers formed therefrom) offer the advantage of facile purification by affinity chromatography over Protein A or Protein G columns.

### Expression Systems

Suitable host cells for expression of Lerk-7 polypeptides include prokaryotes, yeast or higher eukaryotic cells. Appropriate cloning and expression vectors for use with bacterial, fungal, yeast, and mammalian cellular hosts are described, for example, in Pouwels et al. *Cloning Vectors: A Laboratory Manual,* Elsevier, New York, (1985). Cell-free translation systems could also be employed to produce Lerk-7 polypeptides using RNAs derived from DNA constructs disclosed herein.

The expression vector may include DNA encoding a signal or leader peptide fused to the N-terminus of a Lerk-7 polypeptide. The signal or leader peptide co-translationally or post-translationally directs transfer of the Lerk-7 from its site of synthesis to a site inside or outside of the cell membrane or cell wall. The signal or leader peptide is cleaved from the mature Lerk-7 polypeptide. The choice of signal or leader peptide is dependent on the type of host cell that is to be employed.

Suitable prokaryotic host cells for transformation include, for example, *E. coli, Bacillus subtilis, Salmonella typhimurium*, and various other species within the genera *Pseudomonas, Streptomyces*, and *Staphylococcus*. In a prokaryotic host cell, such as *E. coli,* a Lerk-7 polypeptide may include an N-terminal methionine residue to facilitate expression of the recombinant polypeptide in the prokaryotic host cell. The N-terminal Met may be cleaved from the expressed recombinant Lerk-7 polypeptide.

Lerk-7 polypeptides may be expressed in yeast host cells, preferably from the genus *Saccharomyces* (e.g., *S. cerevisiae).* Other genera of yeast, such as *Pichia, K. lactis* or *Kluyveromyces,* may also be employed. Yeast vectors may contain an origin of replication sequence from a 2µ yeast plasmid, an autonomously replicating sequence (ARS), a promoter region, sequences for polyadenylation, sequences for transcription termination, and a selectable marker gene.

Suitable promoter sequences for yeast vectors include, among others, promoters for metallothionein, 3-phosphoglycerate kinase (Hitzeman et al., *J. Biol. Chem.* 255:2073, 1980) or other glycolytic enzymes (Hess et al., *J. Adv. Enzyme Reg.* 7:149, 1968; and Holland et al., *Biochem*. 17:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Another alternative is the glucose-repressible ADH2 promoter described by Russell et al. (*J. Biol. Chem.* 258:2674, 1982) and Beier et al. (*Nature* 300:724, 1982). Other suitable vectors and promoters for use in yeast expression are further described in Hitzeman, EPA-73,657 or in Fleer et. al., *Gene,* 107:285-195 (1991); and van den Berg et. al., *Bio*/*Technology*, 8:135-139 (1990). Shuttle vectors replicable in both yeast and *E. coli* may be constructed by inserting DNA sequences from pBR322 for selection and replication in *E. coli* (Amp^{r} gene and origin of replication) into the above-described yeast vectors.

A suitable leader sequence (e.g. the α-factor leader of *Saccharomyces*) may be employed to direct secretion of the Lerk-7 polypeptide from yeast cells. The α-factor leader sequence is generally inserted between the promoter sequence and the structural gene sequence. See, e.g., Kurjan et al., *Cell* 30:933, 1982; Bitter et al., *Proc. Natl. Acad. Sci. USA* 81:5330, 1984; U. S. Patent 4,546,082; and EP 324,274. Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen et al., *Proc. Natl. Acad. Sci. USA* 75:1929, 1978. The Hinnen et al. protocol selects for Trp⁺ transformants in a selective medium, wherein the selective medium consists of 0.67% yeast nitrogen base. 0.5% casamino acids, 2% glucose, 10 µg/ml adenine and 20 µg/ml uracil.

Yeast host cells transformed by vectors containing ADH2 promoter sequence may be grown for inducing expression in a "rich" medium. An example of a rich medium is one consisting of 1% yeast extract, 2% peptone, and 1% glucose supplemented with 80 µg/ml adenine and 80 µg/ml uracil. Derepression of the ADH2 promoter occurs when glucose is exhausted from the medium.

Mammalian or insect host cell culture systems could also be employed to express recombinant Lerk-7 polypeptides. Baculovirus systems for production of heterologous proteins in insect cells are reviewed by Luckow and Summers, *Bio*/*Technology* 6:47 (1988). Established cell lines of mammalian origin also may be employed. Examples of suitable mammalian host cell lines include the COS-7 line of monkey kidney cells (ATCC CRL 1651; Gluzman et al., *Cell* 23:175, 1981), L cells, C127 cells, 3T3 cells (ATCC CCL 163), Chinese hamster ovary (CHO) cells, HeLa cells, the BHK (ATCC CRL 10) cell line, and the CV-1/EBNA-1 cell line derived from the African green monkey kidney cell line CV 1 (ATCC CCL 70) as described by McMahan et al. (*EMBO J.* 10: 2821, 1991).

Transcriptional and translational control sequences for mammalian host cell expression vectors may be excised from viral genomes. Commonly used promoter sequences and enhancer sequences are derived from Polyoma virus, Adenovirus 2, Simian Virus 40 (SV40), and human cytomegalovirus. DNA sequences derived from the SV40 viral genome, for example, SV40 origin, early and late promoter, enhancer, splice, and polyadenylation sites may be used to provide other genetic elements for expression of a structural gene sequence in a mammalian host cell. Viral early and late promoters are particularly useful because both are easily obtained from a viral genome as a fragment which may also contain a viral origin of replication (Fiers et al., *Nature* 273:113, 1978). Smaller or larger SV40 fragments may also be used, provided the approximately 250 bp sequence extending from the *Hind* III site toward the *Bgl* I site located in the SV40 viral origin of replication site is included.

Examples of expression vectors for use in mammalian host cells can be constructed as disclosed by Okayama and Berg (*Mol. Cell. Biol*. 3:280, 1983). A useful system for stable high level expression of mammalian cDNAs in C127 murine mammary epithelial cells can be constructed substantially as described by Cosman et al. (*Mol*. *Immunol.* 23:935, 1986). A useful high expression vector, PMLSV N1/N4, described by Cosman et al., *Nature* 312:768, 1984 has been deposited as ATCC 39890. Additional useful mammalian expression vectors are described in EP-A-0367566, and in WO 91/18982. The vectors may be derived from retroviruses. In place of the native signal sequence, a heterologous signal sequence may be added, such as the signal sequence for IL-7 described in United States Patent 4.965.195; the signal sequence for IL-2 receptor described in Cosman et al., *Nature* 312:768 (1984); the IL-4 signal peptide described in EP 367,566: the type I IL-1 receptor signal peptide described in U.S. Patent 4,968,607; and the type II IL-1 receptor signal peptide described in EP 460,846.

### Lerk-7 Protein

Lerk-7 polypeptides of the present invention may be produced by recombinant expression systems as described above, or purified from naturally occurring cells. One process for producing Lerk-7 comprises culturing a host cell transformed with an expression vector comprising a DNA sequence that encodes Lerk-7 under conditions sufficient to promote expression of Lerk-7. Lerk-7 is then recovered from the culture medium or cell extracts, depending upon the expression system employed. In one embodiment, a human Lerk-7 protein comprises the amino acid sequence of the protein that is expressed by host cells transformed with an expression vector containing the Lerk-7 cDNA found in strain ATCC 75959.

As is known to the skilled artisan, procedures for purifying a recombinant protein will vary according to such factors as the type of host cells employed and whether or not the recombinant protein is secreted into the culture medium. Other considerations include the types of contaminants that are to be removed, which may vary according to the particular host cells employed to express the desired protein.

For example, when expression systems that secrete the recombinant protein are employed, the culture medium first may be concentrated using a commercially available protein concentration filter, for example, an Amicon or Millipore Pellicon ultrafiltration unit. Following the concentration step, the concentrate can be applied to a purification matrix such as a gel filtration matrix. Alternatively, an anion exchange resin can be employed, for example, a matrix or substrate having pendant diethylaminoethyl (DEAE) groups. The matrices can be acrylamide, agarose, dextran, cellulose or other support materials commonly employed in protein purification. Alternatively, a cation exchange step can be employed. Suitable cation exchangers include various insoluble matrices comprising sulfopropyl or carboxymethyl groups. Sulfopropyl groups are preferred. In addition, one or more reversed-phase high performance liquid chromatography (RP-HPLC) steps employing hydrophobic RP-HPLC media, (e.g., silica gel having pendant methyl or other aliphatic groups) can be employed. Some or all of the foregoing purification steps, in various combinations, may be employed to provide a purified Lerk-7 protein.

A further alternative is affinity chromatography, employing a chromatography matrix containing hek, elk, or an antibody reactive with Lerk-7. The Lerk-7 polypeptides can be recovered from an affinity column using conventional techniques, (e.g., elution in a high salt buffer), then dialyzed into a lower salt buffer for use.

Recombinant protein produced in bacterial culture can be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Finally, RP-HPLC can be employed for final purification steps. Microbial cells can be disrupted by any convenient method, including freeze-thaw cycling, sonication, mechanical disruption, or use of cell lysing agents.

Lerk-7 is preferably expressed as a secreted polypeptide in yeast host cells, in order to simplify purification. Secreted recombinant polypeptide from a yeast host cell fermentation can be purified by methods analogous to those disclosed by Urdal et al. (*J.* *Chromatog*. 296:171, 1984). Urdal et al. describe two sequential, reversed-phase HPLC steps for purification of recombinant human IL-2 on a preparative HPLC column.

The desired degree of purity depends on the intended use of the protein. A relatively high degree of purity is desired when the protein is to be administered *in vivo,* for example. Advantageously, Lerk-7 polypeptides are purified such that no protein bands corresponding to other proteins are detectable upon analysis by SDS-polyacrylamide gel electrophoresis (SDS-PAGE). It will be recognized by one skilled in the pertinent field that multiple bands corresponding to Lerk-7 protein may be visualized by SDS-PAGE, due to differential glycosylation, differential post-translational processing, and the like, as discussed above. A preparation of Lerk-7 protein is considered to be purified as long as no bands corresponding to different (non-Lerk-7) proteins are visualized. Lerk-7 most preferably is purified to substantial homogeneity, as indicated by a single protein band upon analysis by SDS-PAGE.

### Nucleic Acids

The present invention provides isolated Lerk-7 nucleic acids. Such nucleic acids include, but are not limited to, the human Lerk-7 DNA of SEQ ID NO:4, in both single-stranded and double-stranded form, as well as the RNA complement thereof. Lerk-7 DNA of the present invention includes, for example, cDNA, genomic DNA, chemically synthesized DNA, DNA amplified by PCR, and combinations thereof. Genomic DNA may be isolated by conventional techniques using the cDNA isolated in example 1, or a suitable fragment thereof, as a probe.

The present invention further provides fragments of the Lerk-7 nucleotide sequences presented herein. Such fragments desirably comprise at least about 14. preferably at least 17, consecutive nucleotides of the sequence presented in SEQ ID NO:4. DNA and RNA complements of said fragments are provided herein, along with both single-stranded and double-stranded forms of the Lerk-7 DNA.

Among the uses of such Lerk-7 nucleic acid fragments are use as a probe or a primer. Such probes may be employed in cross-species hybridization procedures to isolate Lerk-7 DNA from additional mammalian species. As one example, a probe corresponding to the extracellular domain of a Lerk-7 may be employed. The probes also find use in detecting the presence of Lerk-7 nucleic acids in *in vitro* assays and in such procedures as Northern and Southern blots. Cell types expressing Lerk-7 can be identified. Such procedures are well known, and the skilled artisan can choose a probe of suitable length, depending on the particular intended application. The probes may be labeled (e.g., with ³²P) by conventional techniques.

Oligonucleotides corresponding to segments of Lerk-7 nucleic acids find use as primers, in procedures such as polymerase chain reaction (PCR). For example, 5' and 3' primers corresponding to the termini of a desired Lerk-7 sequence (e.g., a Lerk-7 fragment) are employed to isolate and amplify that sequence in a conventional PCR procedure.

Other useful fragments of the Lerk-7 nucleic acids include antisense or sense oligonucleotides comprising a single-stranded nucleic acid sequence (either RNA or DNA) capable of binding to target Lerk-7 mRNA (sense) or Lerk-7 DNA (antisense) sequences. Antisense or sense oligonucleotides, according to the present invention, comprise a fragment of the coding region of Lerk-7 cDNA. Such a fragment generally comprises at least about 14 nucleotides, preferably from about 14 to about 30 nucleotides. The ability to derive an antisense or a sense oligonucleotide, based upon a cDNA sequence encoding a given protein is described in, for example, Stein and Cohen (*Cancer Res.* 48:2659, 1988) and van der Krol et al. (*BioTechniques* 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of Lerk-7 proteins. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo.* Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

### Antibodies

Antibodies that are immunoreactive with the Lerk-7 polypeptides disclosed herein are provided. Such antibodies specifically bind Lerk-7 in that the antibodies bind to Lerk-7 via the antigen-binding sites of the antibody (as opposed to non-specific binding).

Polyclonal and monoclonal antibodies may be prepared by conventional techniques. See, for example, *Monoclonal Antibodies, Hybridomas: A New Dimension in Biological Analyses,* Kennet et al. (eds.), Plenum Press, New York (1980); and *Antibodies: A Laboratory Manual,* Harlow and Land (eds.), Cold Spring Harbor Laboratory Press. Cold Spring Harbor, NY, (1988). Production of monoclonal antibodies that are immunoreactive with Lerk-7 is further illustrated in example 5 below.

Antigen-binding fragments of such antibodies, which may be produced by conventional techniques, are also encompassed by the present invention. Examples of such fragments include, but are not limited to, Fab, F(ab'), and F(ab')₂ fragments. Antibody fragments and derivatives produced by genetic engineering techniques are also provided.

The monoclonal antibodies of the present invention include chimeric antibodies, e.g., humanized versions of murine monoclonal antibodies. Such humanized antibodies may be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment may comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (*Nature 332*:323, 1988), Liu et al. (*PNAS 84*:3439, 1987), Larrick et al. (*Bio*/*Technology 7*:934, 1989), and Winter and Harris (*TIPS 14*:139, May, 1993).

Among the uses of the antibodies are use in assays to detect the presence of Lerk-7 polypeptides, either *in vitro* or *in vivo.* The antibodies also may be employed in purifying Lerk-7 proteins by immunoaffinity chromatography.

The following examples are provided to further illustrate particular embodiments of the invention, and are not to be construed as limiting the scope of the present invention.

### EXAMPLE 1

### Cloning of Human Lerk-7 cDNA

cDNA encoding a human Lerk-7 of the present invention was isolated by the following procedure. In summary, Lerk-7 DNA was identified during screening of a human fetal brain cDNA library with a probe that was derived from a murine cDNA designated Lerk-6.

Lerk-6 DNA and proteins are described above and in U.S. Patent 5,919,905. The DNA and encoded amino acid sequences for a murine Lerk-6 cDNA are presented herein as SEQ ID NOS: 1 and 2.

A cDNA library consisting of human fetal brain cDNA in the phage vector λgt10 was purchased from Clonetech Laboratories, Inc., Palo Alto, California. A fragment of murine Lerk-6 DNA was isolated by PCR for use as a probe. The Lerk-6 DNA fragment contained nucleotides 11 through 443 of SEQ ID NO:1. A primer employed in the PCR added a T3 RNA polymerase binding site (CTTTAGTGAGGGTTAATCTATAG) (SEQ ID NO:3) to the 3' end of the amplified Lerk-6 DNA fragment.

The probe was labeled with ³²P dCTP using the random priming technique, then allowed to hybridize to pools of cDNA derived from the library. The hybridization was conducted at 55°C overnight in 6 X SSC. The filters were then washed to 0.5X SSC/0.1% SDS at 55°C for about two hours. Hybridizing pools were identified, and the screening was repeated on successively smaller pools, under the same conditions. An individual hybridizing clone, designated clone #16, was purified after the third round of screening.

The DNA sequence of clone #16 was determined. Surprisingly, the clone encoded a novel human protein, designated Lerk-7 herein, rather than encoding the human homolog of Lerk-6. The nucleotide sequence of the coding region of the human Lerk-7 cDNA, and the amino acid sequence encoded thereby, are disclosed in SEQ ID NO:4 and SEQ ID NO:5, respectively. The human Lerk-7 protein of SEQ ID NO:5 comprises an N-terminal signal peptide (amino acids -20 to -1), an extracellular receptor-binding domain (amino acids 1 to 133), a spacer region (amino acids 134 to 183), and a C-terminal hydrophobic region (amino acids 194-208).

Samples of a cell lysate containing a recombinant phage vector (λgt10 containing the human Lerk-7 cDNA of clone #16 inserted into the Eco RI restriction site of the vector) were deposited with the American Type Culture Collection, Rockville, Maryland. The samples were deposited on December 7, 1994, under the terms of the Budapest Treaty, and assigned accession no. ATCC 75959.

### EXAMPLE 2

### Preparation of Soluble elk:Fc Fusion Protein

This example describes construction of an expression vector encoding a soluble elk:Fc fusion protein. This fusion protein can be employed in the binding assays described herein.

A DNA and encoded amino acid sequence for rat elk cDNA is presented in Lhotak et al. (*Mol*. *Cell. Biol. 11*:2496, 1991). The rat elk protein has a 538 amino acid extracellular domain, a 25 amino acid transmembrane domain, and a 419 amino acid cytoplasmic domain.

A rat elk cDNA fragment was fused to the 5' end of cDNA encoding the Fc portion of a human IgGl antibody. An Asp718 restriction endonuclease cleavage site was introduced upstream of the elk coding region. An Asp 718-BgIII fragment of rat elk cDNA (comprising the entire extracellular domain, the transmembrane region, and a small portion of the cytoplasmic domain) was isolated.

DNA encoding a single polypeptide chain comprising the Fc region of a human IgG1 antibody was cloned into the *Spe*I site of pBluescript SK®, a cloning vector that is commercially available from Stratagene Cloning Systems, La Jolla, California. This plasmid vector is replicable in *E. coli* and contains a polylinker segment that includes 21 unique restriction sites. The nucleotide sequence of the cloned DNA, along with the amino acid sequence of the Fc polypeptide encoded thereby, are described in PCT application WO 93/10151, and are also presented in SEQ ID NO:7 and SEQ ID NO:8. A unique *Bgl*II site has been introduced, and encompasses the codons for amino acids three and four of the Fc polypeptide. The encoded Fc polypeptide extends from the N-terminal hinge region to the native C-terminus, i.e., is an essentially full-length antibody Fc region.

The above-described Asp718-BgIII elk cDNA fragment was cloned into the pBluescript SK® vector containing the Fc cDNA, such that the elk cDNA is positioned upstream of the Fc cDNA. Single stranded DNA derived from the resulting gene fusion was mutagenized by the method described in Kunkel (*Proc. Natl. Acad. Sci. USA* 82:488, 1985) and Kunkel et al. (*Methods in Enzymol*. 154:367, 1987) in order to perfectly fuse the entire extracellular domain of elk to the Fc sequence. The mutagenized DNA was sequenced to confirm that the proper nucleotides had been removed (i.e., that the transmembrane region and partial cytoplasmic domain DNA were deleted) and that the elk and Fc sequences were in the same reading frame.

The elk:Fc fusion protein preferably is synthesized in mammalian host cells, such as CV1-EBNA or COS-7 cells. The elk:Fc gene fusion was excised and inserted into a mammalian expression vector designated HAV-EO (Dower et al., *J. Immunol*. 142:4314, 1989). Mammalian host cells were transfected with the resulting recombinant expression vector and cultivated to allow transient expression of the fusion protein, which was secreted into the culture medium via the elk signal peptide. The elk:Fc fusion protein was purified by affinity chromatography, using a protein A sepharose column.

### EXAMPLE 3

### Preparation of Soluble hek:Fc Fusion Protein

This example describes construction of an expression vector encoding a soluble hek:Fc fusion protein. This fusion protein can be employed in the binding assays described herein.

A DNA and encoded amino acid sequence for human hek cDNA is presented in Wicks et al. (*Proc. Natl. Acad. Sci. USA,* 89:1611, 1992), incorporated herein by reference. This hek protein comprises (from N- to C-terminus) an extracellular domain, a transmembrane domain, and a cytoplasmic domain.

Two DNA fragments, one encoding an N-terminal fragment of the extracellular domain of hek and the other encoding a C-terminal fragment of the hek extracellular domain, were isolated by polymerase chain reactions (PCR) conducted under standard conditions, using oligonucleotide primers based on the hek nucleotide sequence published by Wicks et al., *supra.* The template for the PCR was cDNA prepared from mRNA isolated from a human T-cell leukemic cell line designated CCRF-HSB-2 (ATCC CCL-120.1).

The PCR products containing the 5' end of the hek DNA were digested with SpeI and HindIII to isolate a DNA fragment extending from the 5' end of the mature human hek sequence (i.e., lacking DNA encoding the signal sequence) to a HindIII site found in the hek gene. The PCR products containing the 3' end of the hek extracellular domain DNA were digested with HindIII and ClaI to isolate a fragment extending from the internal HindIII site to a ClaI site just downstream of the 3' end of the sequence encoding the hek extracellular domain. The ClaI site is in a multiple cloning site (mcs) introduced just downstream of the extracellular domain.

DNA encoding a mutein of the Fc region of a human IgG1 antibody was isolated. This Fc mutein DNA and the polypeptide encoded thereby are described in U.S. patent 5,457,035, entitled "Novel Cytokine Which is a Ligand for OX40". The mutein DNA was derived from a native Fc polypeptide-encoding DNA by site-directed mutagenesis conducted essentially as described by Deng and Nickoloff, *Anal. Biochem.* 200:81 (1992). The amino acid sequence of the Fc mutein polypeptide is identical to that of the native Fc polypeptide described in PCT application WO 93/10151, except that amino acid 19 has been changed from Leu to Ala, amino acid 20 has been changed from Leu to Glu, and amino acid 22 has been changed from Gly to Ala. This mutein Fc exhibits reduced affinity for immunoglobulin receptors.

A recombinant vector containing the Fc mutein DNA was cleaved with ClaI and NotI, which cleave the vector in a polylinker region immediately upstream and downstream, respectively, of the Fc mutein DNA insert. The desired Fc mutein-encoding fragment was isolated.

A mammalian expression vector designated SMAG4 was cleaved with SpeI and NotI. The SMAG4, vector comprises a murine interleukin-7 signal peptide-encoding sequence (described in U.S. Patent 4,965,195) inserted into the mammalian high expression vector pDC201 (described in Sims et al., *Science* 241:585, 1988, and in PCT application WO 89/03884), which is also capable of replication in *E. coli.* SpeI cleaves the vector immediately downstream of the IL-7 signal peptide-encoding sequence. NotI cleaves approximately 155 bp downstream of the SpeI site in a multiple cloning site of the vector. The large SpeI/NotI fragment containing the vector sequences and the IL-7 signal peptide-encoding DNA was isolated.

A four-way ligation was conducted to insert the two hek-encoding DNA fragments and the Fc mutein-encoding DNA fragment described above into the SpeI/NotI cleaved SMAG4 expression vector. *E. coli* cells were transfected with the ligation mixture and the desired recombinant vector was isolated therefrom. The isolated vector encodes a fusion protein comprising (from N- to C-terminus) the murine IL-7 signal peptide, the hek extracellular domain, four amino acids encoded by the introduced mcs, and the Fc mutein.

The expression vector was then co-transfected with plasmid pSV3.NEO into CV1/EBNA cells. The CV1/EBNA cell line (ATCC CRL 10478) was derived from a monkey kidney cell line as described in McMahan et al. (*EMBO J*., 10:2821, 1991). Vector pSV3.NEO expresses SV40 T-antigen, which is not produced by the host cells. The pSV3.NEO vector is similar to pSV3 (Mulligan and Berg, *Proc. Natl. Acad. Sci. USA* 78:2072, 1981), but additionally contains a neomycin resistance gene. The transformed cells were cultivated to allow transient expression of the fusion protein, which is secreted into the culture medium via the murine IL-7 signal peptide.

### EXAMPLE 4: Binding Study

The binding of Lerk-7 to elk or hek can be assessed by using the following assay. Cells expressing Lerk-7 on the cell surface are prepared. Lerk-7 DNA is amplified by PCR. The primers employed in the PCR are selected to define the termini of the coding region of the Lerk-7 DNA, and also include a Xho I restriction site at the 5' end and a Not I site at the 3' end of the amplified DNA. The 5' primer additionally includes a consensus Kozak sequence upstream of the initiation codon.

The reaction products are digested with Xho I and Not I and inserted into an expression vector pDC410. which is cleaved with Sal I (which is compatible with Xho I) and Not 1. pDC410, a mammalian expression vector that also replicates in *E. coli,* is similar to pDC406 (McMahan et al., *EMBO J. 10*:2821, 1991). The pDC410 multiple cloning site (mcs) differs from that of pDC406 in that it contains additional restriction sites and three stop codons (one in each reading frame). A T7 polymerase promoter downstream of the mcs facilitates sequencing of DNA inserted into the mcs. In addition, the EBV origin of replication is replaced by DNA encoding the SV40 large T antigen (driven from an SV40 promoter) in pDC410.

CV1-EBNA-1 cells in 10 cm² dishes are transfected with the recombinant expression vector containing Lerk-7 DNA. The CV-1/EBNA-1 cell line (ATCC CRL 10478) constitutively expresses EBV nuclear antigen-1 driven from the CMV immediate-early enhancer/promoter. CV1-EBNA-1 is derived from the African Green Monkey kidney cell line CV-1 (ATCC CCL 70), as described by McMahan et al. (*EMBO J.* 10:2821, 1991).

The transfected cells are cultured for 24 hours, and the cells in each dish then are split into a 24-well plate. After culturing an additional 48 hours, a binding assay is conducted by the following procedure. The transfected cells (about 4 x 10⁴ cells/well) are washed with BM-NFDM, which is binding medium (RPMI 1640 containing 25 mg/ml bovine serum albumin. 2 mg/ml sodium azide, 20 mM Hepes pH 7.2) to which 50 mg/ml nonfat dry milk has been added. The cells then are incubated for 1 hour at 37°C with various concentrations of the elk:Fc fusion protein prepared in Example 2 or the hek:Fc fusion protein prepared in Example 3. Cells then are washed and incubated with a constant saturating concentration of a ¹²⁵I-mouse anti-human IgG in binding medium, with gentle agitation for 1 hour at 37°C. After extensive washing, cells are released *via* trypsinization.

The mouse anti-human IgG employed above is directed against the Fc region of human IgG and can be obtained from Jackson Immunoresearch Laboratories, Inc., West Grove, PA. The antibody is radioiodinated using the standard chloramine-T method. The antibody will bind to the Fc portion of any elk:Fc or hek:Fc fusion protein that has bound to the cells. In all assays, non-specific binding of ¹²⁵I-antibody is assayed in the absence of elk:Fc (or hek:Fc), as well as in the presence of elk:Fc (or hek:Fc) and a 200-fold molar excess of unlabeled mouse anti-human IgG antibody.

Cell-bound ¹²⁵I-antibody is quantified on a Packard Autogamma counter. Affinity calculations (Scatchard, *Ann. N.Y. Acad. Sci.* 51:660, 1949) are generated on RS/1 (BBN Software, Boston, MA) run on a Microvax computer.

### EXAMPLE 5

### Monoclonal Antibodies That Bind Lerk-7

This example illustrates a method for preparing monoclonal antibodies that bind Lerk-7. Suitable immunogens that may be employed in generating such antibodies include, but are not limited to, purified Lerk-7 protein or an immunogenic fragment thereof such as the extracellular domain, fusion proteins containing Lerk-7 (e.g., a soluble Lerk-7/Fc fusion protein), or cells expressing Lerk-7 on the cell surface.

Purified Lerk-7 can be used to generate monoclonal antibodies immunoreactive therewith, using conventional techniques such as those described in U.S. Patent 4,411,993. Briefly, mice are immunized with Lerk-7 immunogen emulsified in complete Freund's adjuvant, and injected in amounts ranging from 10-100 µg subcutaneously or intraperitoneally. Ten to twelve days later, the immunized animals are boosted with additional Lerk-7 emulsified in incomplete Freund's adjuvant. Mice are periodically boosted thereafter on a weekly to bi-weekly immunization schedule. Serum samples are periodically taken by retro-orbital bleeding or tail-tip excision to test for Lerk-7 antibodies by dot blot assay, ELISA (Enzyme-Linked Immunosorbent Assay) or inhibition of hek or elk binding.

Following detection of an appropriate antibody titer, positive animals are provided one last intravenous injection of Lerk-7 in saline. Three to four days later, the animals are sacrificed, spleen cells harvested, and spleen cells are fused to a murine myeloma cell line, e.g., NS1 or preferably P3x63Ag8.653 (ATCC CRL 1580). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against purified Lerk-7 by adaptations of the techniques disclosed in Engvall et al., *Immunochem*. 8:871, 1971 and in U.S. Patent 4,703,004. A preferred screening technique is the antibody capture technique described in Beckmann et al., (*J. Immunol*. 144:4212, 1990) Positive hybridoma cells can be injected intraperitoneally into syngeneic BALB/c mice to produce ascites containing high concentrations of anti-Lerk-7 monoclonal antibodies. Alternatively, hybridoma cells can be grown *in vitro* in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to Protein A or Protein G can also be used, as can affinity chromatography based upon binding to Lerk-7.

### EXAMPLE 6

### Southern Blots

Human and murine genomic Southern blots were performed to demonstrate that human Lerk-7 and murine Lerk-6 are not homologues. Probing human DNA with human Lerk-7 or murine Lerk-6 resulted in the following hybridizing bands (in kbp): Lerk-7 [EcoR1 (12.5, 9, 6, 4, 2.5) and BamH1 (13 and 9)]; Lerk-6 [EcoR1 (5) and Bam H1 (5.3)]. Probing murine genomic DNA results in the following hybridizing bands: Lerk-7 [EcoR1 (12.5, 6) and BamH 1 (18, 6)]; Lerk-6 [EcoR1 (18) and BamH1 (2.7, 2.2). Since the Lerk-7 and Lerk-6 hybridizing bands are not identical, these cDNAs define unique genes.

### EXAMPLE 7: Binding Study

The binding affinity of Lerk-7 for elk or for hek was determined in the following assay. cDNA encoding the full length Lerk-7 polypetide of SEQ ID NO:5 was inserted into SF CAV, a mammalian expression vector that also replicates in *E. coli.* The SF CAV vector is described in PCT application WO 93/19777.

CV-1 EBNA-1 cells (described in example 3) in 10 cm² dishes were transfected with the recombinant expression vector containing Lerk-7 DNA. The transfected cells were cultured for 24 hours, and the cells in each dish then were split into a 24-well plate. After culturing an additional 48 hours, a binding assay was conducted by the following procedure. The transfected cells (about 4 x 10⁴ cells/well) were washed with BM-NFDM, which is binding medium (RPMI 1640 containing 25 mg/ml bovine serum albumin, 2 mg/ml sodium azide, and 20 mM Hepes pH 7.2) to which 50 mg/ml nonfat dry milk has been added. The cells then were incubated for I hour at 37°C with various concentrations of a soluble elk:Fc or hek:Fc fusion protein. The hek:Fc fusion protein was prepared as described in example 3. The elk:Fc fusion protein was prepared by procedures analogous to those described in example 2, except that the mutein Fc polypeptide described in example 3 was substituted for the native Fc polypeptide.

Cells then were washed and incubated with a constant saturating concentration (20 ng/ml) of a ¹²⁵I-mouse anti-human IgG in binding medium, with gentle agitation for 1 hour at 37°C. After extensive washing, the cells were harvested by trypsinization.

The mouse anti-human IgG employed above is directed against the Fc region of human IgG and is available from Jackson Immunoresearch Laboratories, Inc., West Grove, PA. The antibody is radioiodinated using the standard chloramine-T method. The antibody will bind to the Fc portion of any elk:Fc or hek:Fc fusion protein that has bound to the cells. In all assays, non-specific binding of ¹²⁵I-antibody was assayed in the absence of elk:Fc (or hek:Fc), as well as in the presence of elk:Fc (or hek:Fc) and a 200-fold molar excess of unlabeled mouse anti-human IgG antibody.

Cell-bound ¹²⁵I-antibody was quantified on a Packard Autogamma counter. Affinity calculations (Scatchard, *Ann. N.Y. Acad. Sci.* 51:660, 1949) were generated on RS/1 (BBN Software, Boston, MA) run on a Microvax computer. The results were as follows.

Binding of elk:Fc to the cells expressing Lerk-7 was characterized by a biphasic pattern, with affinity constants (Kₐ) calculated to be Kₐ₁ = 1.06 x 10⁸ and Kₐ₂ = 4.95 x 10⁷. Binding of hek:Fc exhibited a single affinity class of binding (Kₐ = 5.0 x 10⁸).

### BRIEF DESCRIPTION OF THE SEQUENCE LISTING

SEQ ID NO: 1 and SEQ ID NO:2 present the DNA sequence of a cloned cDNA encoding a protein designated Lerk-6, and the amino acid sequence encoded thereby, respectively. A fragment of the DNA of SEQ ID NO:1 was used as a probe to isolate the Lerk-7 DNA of the present invention, as described in example 1.
SEQ ID NO:3 presents the DNA sequence of a T3 RNA polymerase binding site, as described in example 1.
SEQ ID NO:4 and SEQ ID NO:5 present the DNA sequence of a cloned human Lerk-7 cDNA, and the amino acid sequence encoded thereby, respectively. Isolation of this cDNA is described in example 1.
SEQ ID NO:6 presents the amino acid sequence of the Flag® peptide.
SEQ ID NO:7 and SEQ ID NO:8 present the DNA and encoded amino acid sequences, respectively, of a cloned cDNA encoding the Fc region of a human IgG1 antibody.

## Claims

1. An isolated DNA encoding a Lerk-7 polypeptide that binds hek or elk, wherein said Lerk-7 polypeptide comprises an amino acid sequence that is at least 80% identical to a sequence selected from the group consisting of residues -20 to 208 of SEQ ID NO:5 and residues 1 to 208 of SEQ ID NO:5.

2. A DNA of claim 1, wherein said Lerk-7 polypeptide comprises an amino acid sequence that is at least 90% identical to a sequence selected from the group consisting of residues -20 to 208 of SEQ ID NO:5 and residues 1 to 208 of SEQ ID NO:5.

3. A DNA of claim 2, wherein said Lerk-7 polypeptide comprises an amino acid sequence selected from the group consisting of residues -20 to 208 of SEQ ID NO:5 and residues 1 to 208 of SEQ ID NO:5.

4. A DNA of claim 3, comprising a nucleotide sequence selected from the group consisting of nucleotides 1-687 of SEQ ID NO:4 and nucleotides 61-687 of SEQ ID NO:4.

5. An isolated DNA encoding a soluble Lerk-7 polypeptide that binds hek or elk, wherein said Lerk-7 polypeptide comprises an amino acid sequence that is at least 80% identical to a sequence selected from the group consisting of residues -20 to x of SEQ ID NO:5 and residues 1 to x of SEQ ID NO:5, wherein x represents an integer between 133 and 193, inclusive.

6. An isolated DNA according to claim 5, wherein said Lerk-7 polypeptide comprises an amino acid sequence selected from the group consisting of residues -20 to x of SEQ ID NO:5 and residues 1 to x of SEQ ID NO:5, wherein x represents an integer between 133 and 193, inclusive.

7. A DNA of claim 6, wherein said Lerk-7 polypeptide comprises an amino acid sequence selected from the group consisting of residues -20 to 133, -20 to 182, -20 to 193, 1 to 133, 1 to 182 and 1 to 193 of SEQ ID NO:5.

8. An expression vector comprising a DNA sequence according to any of claims 1 to 7.

9. A host cell transformed with an expression vector of claim 8.

10. A process for producing a Lerk-7 polypeptide, comprising culturing a host cell of claim 9 under conditions that promote expression of said Lerk-7 polypeptide, and recovering the Lerk-7 polypeptide.

11. A purified Lerk-7 polypeptide encoding by a DNA according to any of claims 1 to 7.

12. A purified Lerk-7 polypeptide comprising an amino acid sequence that is at least 80% identical to the sequence of amino acid residues 1 to 208 of SEQ ID NO:5.

13. A Lerk-7 polypeptide of claim 12, comprising the sequence of amino acid residues 1 to 208 of SEQ ID NO:5.

14. A purified soluble Lerk-7 polypeptide that binds hek or elk, wherein said polypeptide comprises an amino acid sequence that is at least 80% identical to the sequence of residues 1 to x of SEQ ID NO:5, wherein x represents an integer between 133 and 193, inclusive.

15. A soluble Lerk-7 polypeptide according to claim 14, wherein said polypeptide comprises the amino acid sequence of residues 1 to x of SEQ ID NO:5, wherein x represents an integer between 133 and 193, inclusive.

16. A soluble Lerk-7 polypeptide according to claim 15, comprising an amino acid sequence selected from the group consisting of residues 1 to 133, 1 to 182, and 1 to 193 of SEQ ID NO:5.

17. A purified Lerk-7 polypeptide which is a fragment of the Lerk-7 protein of SEQ ID NO:5, wherein said fragment binds hek or elk.

18. A purified Lerk-7 polypeptide **characterized by** an N-terminal amino acid sequence Gln-Asp-Pro-Gly-Ser-Lys-Ala-Val-Ala-Asp-Arg-Tyr-Ala-Val-Tyr-.

19. A purified Lerk-7 protein comprising the amino acid sequence of the protein expressed from the Lerk-7 cDNA insert of the recombinant vector in ATCC 75959.

20. An antibody that specifically binds a Lerk-7 polypeptide according to any of claims 11 to 19, or an antigen-binding fragment thereof.

21. An antibody according to claim 20, wherein the antibody is a monoclonal antibody.

22. A monoclonal antibody according to claim 21, wherein said antibody specifically binds the Lerk-7 polypeptide of SEQ ID NO:5.

23. A fusion protein comprising a soluble Lerk-7 polypeptide and an antibody-derived polypeptide, wherein said Lerk-7 polypeptide is a soluble fragment of the Lerk-7 protein of SEQ ID NO:5, wherein said fragment is capable of binding elk or hek.

24. A fusion protein of claim 23, wherein said antibody-derived polypeptide is an Fc polypeptide.

25. A dimer comprising two fusion proteins of claim 24.

26. An oligomer comprising from two to four soluble Lerk-7 polypeptides, wherein each of said polypeptides is a soluble fragment of the Lerk-7 protein of SEQ ID NO:5, wherein said fragment is capable of binding elk or hek.

27. A composition comprising a Lerk-7 polypeptide or oligomer according to any of claims 11-19, 25 or 26, and a pharmaceutically acceptable diluent, carrier, or excipient.

28. A conjugate comprising a Lerk-7 polypeptide or oligomer according to any of claims 11-19, 25 or 26, and a diagnostic or therapeutic agent.

29. A Lerk-7 polypeptide, oligomer, composition, or conjugate according to any of claims 11-19, 25-28 for use in human medicine.

30. The use of a Lerk-7 polypeptide, oligomer, or composition according to any of claims 11-19 or 25-27 in the preparation of a medicament for treating a disorder of neural tissue.

31. The use of a Lerk-7 polypeptide, oligomer, or composition according to any of claims 11-19 or 25-27 for detecting or binding a receptor that binds Lerk-7.

## Patentansprüche

1. Isolierte DNA, die ein LERK-7-Polypeptid codiert, das HEK oder ELK bindet, wobei das LERK-7-Polypeptid eine Aminosäuresequenz aufweist, die wenigstens zu 80 % mit einer Sequenz identisch ist, die aus der Gruppe ausgewählt ist, die aus Resten von -20 bis 208 der SEQ ID NO: 5 und Resten von 1 bis 208 der SEQ ID NO: 5 besteht.

2. DNA nach Anspruch 1, wobei das LERK-7-Polypeptid eine Aminosäuresequenz aufweist, die wenigstens zu 90 % mit einer Sequenz identisch ist, die aus der Gruppe ausgewählt ist, die aus Resten von -20 bis 208 der SEQ ID NO: 5 und Resten von 1 bis 208 der SEQ ID NO: 5 besteht.

3. DNA nach Anspruch 2, wobei das LERK-7-Polypeptid eine Aminosäuresequenz aufweist, die aus der Gruppe ausgewählt ist, die aus Resten von -20 bis 208 der SEQ ID NO: 5 und Resten von 1 bis 208 der SEQ ID NO: 5 besteht.

4. DNA nach Anspruch 3, die eine Nucleotidsequenz aufweist, die aus der Gruppe ausgewählt ist, die aus Nucleotiden 1-687 der SEQ ID NO: 4 und Nucleotiden 61-687 der SEQ ID NO: 4 besteht.

5. Isolierte DNA, die ein lösliches LERK-7-Polypeptid codiert, das HEK oder ELK bindet, wobei das LERK-7-Polypeptid eine Aminosäuresequenz aufweist, die wenigstens zu 80 % mit einer Sequenz identisch ist, die aus der Gruppe ausgewählt ist, die aus Resten von -20 bis x der SEQ ID NO: 5 und Resten von 1 bis x der SEQ ID NO: 5 besteht, wobei x eine ganze Zahl zwischen einschließlich 133 und einschließlich 193 repräsentiert.

6. Isolierte DNA nach Anspruch 5, wobei das LERK-7-Polypeptid eine Aminosäuresequenz aufweist, die aus der Gruppe ausgewählt ist, die aus Resten von -20 bis x der SEQ ID NO: 5 und Resten von 1 bis x der SEQ ID NO: 5 besteht, wobei x eine ganze Zahl zwischen einschließlich 133 und einschließlich 193 repräsentiert.

7. DNA nach Anspruch 6, wobei das LERK-7-Polypeptid eine Aminosäuresequenz aufweist, die aus der Gruppe ausgewählt ist, die aus Resten von -20 bis 133, -20 bis 182, -20 bis 193, 1 bis 133, 1 bis 182 und 1 bis 193 der SEQ ID NO: 5 besteht.

8. Expressionsvektor, der eine DNA-Sequenz nach einem der Ansprüche 1 bis 7 aufweist.

9. Wirtszelle, die mit einem Expressionsvektor nach Anspruch 8 transformiert ist.

10. Verfahren zur Herstellung eines LERK-7-Polypeptids, das Kultivieren einer Wirtszelle nach Anspruch 9 unter Bedingungen, die eine Expression des LERK-7-Polypeptids begünstigen, und ein Gewinnen des LERK-7-Polypeptids aufweist.

11. Gereinigtes LERK-7-Polypeptid, das durch eine DNA nach einem der Ansprüche 1 bis 7 codiert ist.

12. Gereinigtes LERK-7-Polypeptid, das eine Aminosäuresequenz aufweist, die wenigstens zu 80 % mit der Sequenz der Aminosäurereste von 1 bis 208 der SEQ ID NO: 5 identisch ist.

13. LERK-7-Polypeptid nach Anspruch 12, wobei die Sequenz die Aminosäurereste von 1 bis 208 der SEQ ID NO: 5 aufweist.

14. Gereinigtes, lösliches LERK-7-Polypeptid, das HEK oder ELK bindet, wobei das Polypeptid eine Aminosäuresequenz aufweist, die wenigstens zu 80 % mit der Sequenz der Reste von 1 bis x der SEQ ID NO: 5 identisch ist, wobei x eine ganze Zahl zwischen einschließlich 133 und einschließlich 193 repräsentiert.

15. Lösliches LERK-7-Polypeptid nach Anspruch 14, wobei das Polypeptid die Aminosäuresequenz der Reste von 1 bis x der SEQ ID NO: 5 aufweist, wobei x eine ganze Zahl zwischen einschließlich 133 und einschließlich 193 repräsentiert.

16. Lösliches LERK-7-Polypeptid nach Anspruch 15, das eine Aminosäuresequenz aufweist, die aus der Gruppe ausgewählt ist, die aus den Resten von 1 bis 133, 1 bis 182 und 1 bis 193 der SEQ ID NO: 5 besteht.

17. Gereinigtes LERK-7-Polypeptid, das ein Fragment des LERK-7-Proteins der SEQ ID NO: 5 ist, wobei das Fragment HEK oder ELK bindet.

18. Gereinigtes LERK-7-Polypeptid, **gekennzeichnet durch** eine N-tenninale Aminosäuresequenz Gln-Asp-Pro-Gly-Ser-Lys-Ala-Val-Ala-Asp-Arg-Tyr-Ala-Val-Tyr-.

19. Gereinigtes LERK-7-Protein, das die Aminosäuresequenz des von dem LERK-7-cDNA-Insert des rekombinanten Vektors in ATCC 75 959 exprimierten Proteins aufweist.

20. Antikörper, der spezifisch ein LERK-7-Polypeptid nach einem der Ansprüche 11 bis 19 oder ein Antigenbindungsfragment davon bindet.

21. Antikörper nach Anspruch 20, wobei der Antikörper ein monoklonaler Antikörper ist.

22. Monoklonaler Antikörper nach Anspruch 21, wobei der Antikörper spezifisch das LERK-7-Polypeptid der SEQ ID NO: 5 bindet.

23. Fusionsprotein, das ein lösliches LERK-7-Polypeptid und ein von einem Antikörper abgeleitetes Polypeptid aufweist, wobei das LERK-7-Polypeptid ein lösliches Fragment des LERK-7-Proteins der SEQ ID NO: 5 ist, wobei das Fragment fähig ist, ELK oder HEK zu binden.

24. Fusionsprotein nach Anspruch 23, wobei das von einem Antikörper abgeleitete Polypeptid ein Fc-Polypeptid ist.

25. Dimer, welches zwei Fusionsproteine nach Anspruch 24 aufweist.

26. Oligomer, das zwei bis vier lösliche LERK-7-Polypeptide aufweist, wobei jedes der Polypeptide ein lösliches Fragment des LERK-7-Proteins der SEQ ID NO: 5 ist, wobei das Fragment fähig ist, ELK oder HEK zu binden.

27. Zusammensetzung, die ein LERK-7-Polypeptid oder -Oligomer nach einem der Ansprüche 11 bis 19, 25 oder 26 und ein für Pharmazeutika zugelassenes Verdünnungs-, Träger- oder Bindemittel aufweist.

28. Konjugat, das ein LERK-7-Polypeptid oder -Oligomer nach einem der Ansprüche 11 bis 19, 25 oder 26 und ein Diagnostikum oder Therapeutikum aufweist.

29. LERK-7-Polypeptid, -Oligomer, -Zusammensetzung oder -Konjugat nach einem der Ansprüche 11 bis 19, 25 bis 28 zur Verwendung in der Humanmedizin.

30. Verwendung eines LERK-7-Polypeptids, -Oligomers oder einer LERK-7-Zusammensetzung nach einem der Ansprüche 11 bis 19 oder 25 bis 27 zur Herstellung eines Medikaments zur Behandlung einer Erkrankung von Nervengewebe.

31. Verwendung eines LERK-7-Polypeptids, -Oligomers oder einer LERK-7-Zusammensetzung nach einem der Ansprüche 11 bis 19 oder 25 bis 27 zur Auffindung oder Bindung eines Rezeptors, der LERK-7 bindet.

## Revendications

1. ADN isolé codant pour un polypeptide Lerk-7 qui lie hek ou elk, dans lequel ledit polypeptide Lerk-7 comprend une séquence d'acides aminés qui est au moins identique à 80% à une séquence sélectionnée parmi le groupe comprenant les résidus -20 à 208 de la SEQ ID NO:5 et les résidus 1 à 208 de la SEQ ID NO:5.

2. ADN suivant la revendication 1, dans lequel ledit polypeptide Lerk-7 comprend une séquence d'acides aminés qui est au moins identique à 90% à une séquence sélectionnée parmi le groupe comprenant les résidus -20 à 208 de la SEQ ID NO:5 et les résidus 1 à 208 de la SEQ ID NO:5.

3. ADN suivant la revendication 2, dans lequel ledit polypeptide Lerk-7 comprend une séquence d'acides aminés sélectionnée parmi le groupe comprenant les résidus -20 à 208 de la SEQ ID NO:5 et les résidus 1 à 208 de la SEQ ID NO:5.

4. ADN suivant la revendication 3, comprenant une séquence de nucléotides sélectionnée parmi le groupe comprenant les nucléotides 1-687 de la SEQ ID NO:4 et les nucléotides 61-687 de la SEQ ID NO:4.

5. ADN isolé codant pour un polypeptide Lerk-7 soluble qui lie hek ou elk, dans lequel ledit polypeptide Lerk-7 comprend une séquence d'acides aminés qui est au moins identique à 80% à une séquence sélectionnée parmi le groupe comprenant les résidus -20 à x de la SEQ ID NO:5 et les résidus 1 à x de la SEQ ID NO:5, x représentant un entier entre 133 et 193 compris.

6. ADN isolé suivant la revendication 5, dans lequel ledit polypeptide Lerk-7 comprend une séquence d'acides aminés sélectionnée parmi le groupe comprenant les résidus -20 à x de la SEQ ID NO:5 et les résidus 1 à x de la SEQ ID NO:5, x représentant un entier entre 133 et 193 compris.

7. ADN suivant la revendication 6, dans lequel ledit polypeptide Lerk-7 comprend une séquence d'acides aminés sélectionnée parmi le groupe comprenant les résidus -20 à 133, -20 à 182, -20 à 193, 1 à 133, 1 à 182 et 1 à 193 de la SEQ ID NO:5.

8. Vecteur d'expression comprenant une séquence d'ADN suivant l'une quelconque des revendications 1 à 7.

9. Cellule hôte transformée par un vecteur d'expression suivant la revendication 8.

10. Procédé de production d'un polypeptide Lerk-7, comprenant la mise en culture d'une cellule hôte suivant la revendication 9 dans des conditions qui promeuvent l'expression dudit polypeptide Lerk-7, et la récupération du polypeptide Lerk-7.

11. Polypeptide Lerk-7 purifié codé par un ADN suivant l'une quelconque des revendications 1 à 7.

12. Polypeptide Lerk-7 purifié comprenant une séquence d'acides aminés qui est au moins identique à 80% à la séquence de résidus acides aminés 1 à 208 de la SEQ ID NO:5.

13. Polypeptide Lerk-7 suivant la revendication 12, comprenant la séquence de résidus acides aminés 1 à 208 de la SEQ ID NO:5.

14. Polypeptide Lerk-7 soluble purifié qui lie hek ou elk, dans lequel ledit polypeptide comprend une séquence d'acides aminés qui est au moins identique à 80% à la séquence de résidus 1 à x de la SEQ ID NO:5, x représentant un entier entre 133 et 193 compris.

15. Polypeptide Lerk-7 soluble suivant la revendication 14, dans lequel ledit polypeptide comprend la séquence d'acides aminés des résidus 1 à x de la SEQ ID NO:5, x représentant un entier entre 133 et 193 compris.

16. Polypeptide Lerk-7 soluble suivant la revendication 15, comprenant une séquence d'acides aminés sélectionnée parmi le groupe comprenant les résidus 1 à 133, 1 à 182, et 1 à 193 de la SEQ ID NO:5.

17. Polypeptide Lerk-7 purifié qui est un fragment de la protéine Lerk-7 de la SEQ ID NO:5, dans lequel ledit fragment lie hek ou elk.

18. Polypeptide Lerk-7 purifié **caractérisé par** une séquence d'acides aminés en extrémité N Gln-Asp-Pro-Gly-Ser-Lys-Ala-Val-Ala-Asp-Arg-Tyr-Ala-Val-Tyr-.

19. Protéine Lerk-7 purifiée comprenant la séquence d'acides aminés de la protéine exprimée à partir de l'insert d'ADNc de Lerk-7 du vecteur recombinant dans l'ATCC 75 959.

20. Anticorps qui lie spécifiquement un polypeptide Lerk-7 suivant l'une quelconque des revendications 11 à 19, ou un fragment liant l'antigène de celui-ci.

21. Anticorps suivant la revendication 20, dans lequel l'anticorps est un anticorps monoclonal.

22. Anticorps monoclonal suivant la revendication 21, dans lequel ledit anticorps lie spécifiquement le polypeptide Lerk-7 de la SEQ ID NO:5.

23. Protéine de fusion comprenant un polypeptide Lerk-7 soluble et un polypeptide provenant d'un anticorps, dans laquelle ledit polypeptide Lerk-7 est un fragment soluble de la protéine Lerk-7 de la SEQ ID NO: 5, ledit fragment pouvant lier elk ou hek.

24. Protéine de fusion suivant la revendication 23, dans laquelle ledit polypeptide provenant d'un anticorps est un polypeptide Fc.

25. Dimère comprenant deux protéines de fusion suivant la revendication 24.

26. Oligomère comprenant de deux à quatre polypeptides Lerk-7 solubles, dans lequel chacun desdits polypeptides est un fragment soluble de la protéine Lerk-7 de la SEQ ID NO:5, ledit fragment pouvant lier elk ou hek.

27. Composition comprenant un polypeptide Lerk-7 ou un oligomère suivant l'une quelconque des revendications 11-19, 25 ou 26, et un diluant, vecteur ou excipient pharmaceutiquement acceptable.

28. Conjugué comprenant un polypeptide Lerk-7 ou un oligomère suivant l'une quelconque des revendications 11-19, 25 ou 26, et un agent de diagnostic ou thérapeutique.

29. Polypeptide Lerk-7, oligomère, composition ou conjugué suivant l'une quelconque des revendications 11-19, 25-28 pour une utilisation en médecine humaine.

30. Utilisation d'un polypeptide Lerk-7, d'un oligomère ou d'une composition suivant l'une quelconque des revendications 11-19 ou 25-27 dans la préparation d'un médicament pour le traitement d'un trouble du tissu neural.

31. Utilisation d'un polypeptide Lerk-7, d'un oligomère ou d'une composition suivant l'une quelconque des revendications 11-19 ou 25-27 pour la détection ou la liaison d'un récepteur qui lie Lerk-7.
